(19) Europäisches Patentamt / European Patent Office / Office européen des brevets

(11) **EP 1 988 482 A2**

(12) **EUROPEAN PATENT APPLICATION**

(43) Date of publication:
05.11.2008 Bulletin 2008/45

(51) Int Cl.:
*G06F 19/00* (2006.01)  *A61B 5/00* (2006.01)

(21) Application number: 08251030.6

(22) Date of filing: 20.03.2008

| | |
|---|---|
| (84) Designated Contracting States:<br>AT BE BG CH CY CZ DE DK EE ES FI FR GB GR HR HU IE IS IT LI LT LU LV MC MT NL NO PL PT RO SE SI SK TR<br>Designated Extension States:<br>AL BA MK RS | • **Rodbard, David**<br>**Potomac, Maryland 20854 (US)**<br>• **Price, David**<br>**Pleasanton, California 94566 (US)** |
| (30) Priority: 20.03.2007 US 688743 | (74) Representative: **Tunstall, Christopher Stephen et al**<br>**Carpmaels & Ransford**<br>**43-45 Bloomsbury Square**<br>**London WC1A 2RA (GB)** |
| (71) Applicant: **Lifescan, Inc.**<br>**Milpitas, CA 95035 (US)** | |
| (72) Inventors:<br>• **Ray, Pinaki**<br>**Fremont, California 94539 (US)**<br>• **Matian, Greg**<br>**Foster City, California 94404 (US)**<br>• **Srinivasan, Aparna**<br>**San Jose, California 95136 (US)** | Remarks:<br>A request for correction of the drawings has been filed pursuant to Rule 139 EPC. A decision on the request will be taken during the proceedings before the Examining Division (Guidelines for Examination in the EPO, A-V, 3.). |

(54) **Computer program for diabetes management**

(57) A diabetes management system or process is provided herein that may be used to analyze and recognize patterns for a large amount of blood glucose concentration measurements and other physiological parameters related to the glycemia of a patient. In particular, a method of monitoring glycemia in a patient may include storing a patient's data on a suitable device, such as, for example, a glucose meter. The patient's data may include blood glucose concentration measurements. The diabetes management system or process may be installed on, but is not limited to, a personal computer, an insulin pen, an insulin pump, or a glucose meter. The diabetes management system or process may identify a plurality of pattern types from the data including a testing/dosing pattern, a hypoglycemic pattern, a hyperglycemic pattern, a variability pattern, and a comparative pattern. After identifying a particular pattern with the data management system or process, a warning message may be displayed on a screen of a personal computer or a glucose meter. Other messages can also be provided to ensure compliance of any prescribed diabetes regiments or to guide the patient in managing the patient's diabetes.

FIG. 2

EP 1 988 482 A2

## Description

This application claims the benefits of U.S. Patent Application Serial No. 11/688,629, filed on March 20, 2007, which application is hereby incorporated by reference in its entirety into this application.

People with diabetes often rely upon the use of blood glucose meter in conjunction with help from their physicians for managing their disease. In addition, people with diabetes typically use a logbook to keep track of their glucose concentration measurements. Under certain circumstances, interpreting a large number of glucose concentration measurements in a logbook format can be difficult, complex, and time consuming. To further complicate matters, physicians usually have limited time constraints in assisting people with diabetes to interpret a large number of glucose concentration measurements. When such complication with blood glucose values is further compounded by the need to assess the effect of insulin or type of insulin, and other physiological parameters or external parameters, it is believed that the task of the clinician, physician or person with diabetes is made even more difficult. An additional hurdle for physicians or clinicians is the time constraint placed upon an office visit for the patient due to the economics of running a medical office. It is believed that in most cases, a physician or clinician typically spends less than approximately seven (7) minutes per patient, which results in little or no time for assessment or guidance for the patient. Applicants have recognized the need to allow for simple and quick assessment of glycemic trends, patterns, data, and graphical correlation of important blood glucose and other physiological or external parameters by a busy physician, clinician, and the patient.

## SUMMARY

In one aspect, a diabetes management system or process is provided herein that may be used to analyze and recognize patterns for a large number of glucose concentration measurements and other physiological or external parameters related to the glycemia of a patient. In particular, a method of monitoring glycemia in a patient may include storing a patient's data on a suitable device, such as, for example, a glucose meter. The patient's data may include blood glucose concentration measurements. The diabetes management system or process may be installed on, but is not limited to, a personal computer, an insulin pen, an insulin pump, or a glucose meter. The diabetes management system or process may identify a plurality of pattern types from the data including a testing/dosing pattern, a hypoglycemic pattern, a hyperglycemic pattern, a blood glucose variability pattern, and a comparative pattern. After identifying a particular pattern with the data management system or process, a warning message may be displayed on a screen of a personal computer or a glucose meter. Other messages can also be provided to ensure compliance of any prescribed diabetes regiments or to guide the patient in managing the patient's diabetes.

In particular, a computer program to provide diabetes management information to a user is provided. The computer program includes a user interface, database module, business object module, and a diabetes management rules engine. The database module has a plurality of data inputs relating to blood glucose of a patient. The business object module is in communication with the database module. The diabetes management rules engine generates a plurality of textual messages to the user interface based on the database module and the business object module. The textual messages include statistically significant patterns from the patient's data indicative of at least excessive variability by at least one of time of day, by day in a week, both by time of day and day of week, or at different time intervals.

In yet another embodiment, a method of monitoring glycemia in a patient is provided. The method can be achieved by storing a patient's data that includes blood glucose concentration measurements; generating a plurality of patterns including multiple blood glucose variability patterns; and displaying at least one of the multiple blood glucose variability patterns over a predetermined time period.

In a further embodiment, a method of monitoring glycemia in a patient is provided. The method can be achieved by storing a patient's data that includes blood glucose concentration measurements; generating statistically significant patterns from the patient's data, the patterns indicative of hypoglycemia, hyperglycemia, or excessive blood glucose variability by time of day, by day in a week, both by time of day and day of week, or at different time intervals; calculating standard error and Z test with data from the table; and displaying a message upon the Z test is greater than a predetermined value indicative of a pattern of glycemia outside at least a predetermined range for such pattern.

These and other embodiments, features and advantages will become apparent to those skilled in the art when taken with reference to the following more detailed description of the invention in conjunction with the accompanying drawings that are first briefly described.

## BRIEF DESCRIPTION OF THE DRAWINGS

The accompanying drawings, which are incorporated herein and constitute part of this specification, illustrate presently preferred embodiments of the invention, and, together with the general description given above and the detailed description given below, serve to explain features of the invention (wherein like numerals represent like elements), of which:

Figure 1 illustrates a schematic of a diabetes management system that includes a glucose meter, an insulin pump, and a personal computer;

Figure 2 illustrates a schematic of a diabetes management rules engine;

Figures 3A and 3B illustrate a flow chart of the diabetes management system or process system;

Figure 4A illustrates a flow chart for analyzing a plurality of glucose concentration measurements for a pattern that may indicate an incidence of hypoglycemia;

Figure 4B illustrates a flow chart for analyzing a plurality of glucose concentration measurements for a pattern that may indicate a hypoglycemic pattern by time slot;

Figure 4C illustrates an exemplary chi-squared table that can be used to determine statistically significant patterns based on a patient's data.

Figure 5 illustrates a flow chart for analyzing a plurality of glucose concentration measurements for a pattern that may indicate a hypoglycemic pattern by day of week;

Figure 6 illustrates a flow chart for analyzing a plurality of glucose concentration measurements for a pattern that may indicate an incidence of hypoglycemia by meal;

Figure 7A illustrates a flow chart for analyzing a plurality of glucose concentration measurements for a pattern that may indicate an incidence of hyperglycemia;

Figure 7B illustrates a flow chart for analyzing a plurality of glucose concentration measurements for a pattern that may indicate a hyperglycemic pattern by time slot;

Figure 8 illustrates a flow chart for analyzing a plurality of glucose concentration measurements for a pattern that may indicate a hyperglycemic pattern by day of week;

Figure 9 illustrates a flow chart for analyzing a plurality of glucose concentration measurements for a pattern that may indicate an incidence of hyperglycemia by meal;

Figure 10 illustrates a flow chart for analyzing a plurality of glucose concentration measurements for a pattern that may indicate an incidence of high blood glucose variability;

Figure 11 illustrates a flow chart for analyzing a plurality of glucose concentration measurements for a pattern that may indicate an overcorrection for hypoglycemia;

Figure 12 illustrates a flow chart for analyzing a plurality of glucose concentration measurements for a pattern that may indicate an overcorrection for hyperglycemia;

Figure 13 illustrates a flow chart for analyzing a plurality of glucose concentration measurements for a pattern that may indicate whether the frequency of glucose testing is sufficient;

Figure 14A illustrates a flow chart for analyzing a plurality of glucose concentration measurements for a pattern that may indicate whether the adequacy of pre-meal testing is sufficient;

Figure 14B illustrates a flow chart for analyzing a plurality of glucose concentration measurements for a pattern that may indicate whether the adequacy of post-meal testing is sufficient;

Figure 14C illustrates a flow chart for analyzing a plurality of glucose concentration measurements for determining whether a user is complying with a targeted testing frequency;

Figure 15 illustrates a flow chart for analyzing a plurality of glucose concentration measurements for comparing pre-meal and post-meal glucose concentrations;

Figure 16 illustrates a flow chart for analyzing a plurality of glucose concentration measurements for comparing daytime and nighttime glucose concentrations;

Figure 17A illustrates a flow chart for analyzing a plurality of glucose concentration measurements for comparing previous and current hypoglycemic incidence;

Figure 17B illustrates a flow chart for analyzing a plurality of glucose concentration measurements for comparing previous and current hyperglycemic incidence;

Figure 18 illustrates a summary report by time of day generated using an embodiment of the diabetes management system; and

Figures 19A and 19B illustrate a patient information sheet that was generated using an embodiment of the diabetes management system.

## DETAILED DESCRIPTION OF ILLUSTRATIVE EMBODIMENTS

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will enable one skilled in the art to make and use the invention, and describes several embodiments, adaptations, variations, alternatives and uses of the invention, including what is presently believed to be the best mode of carrying out the invention.

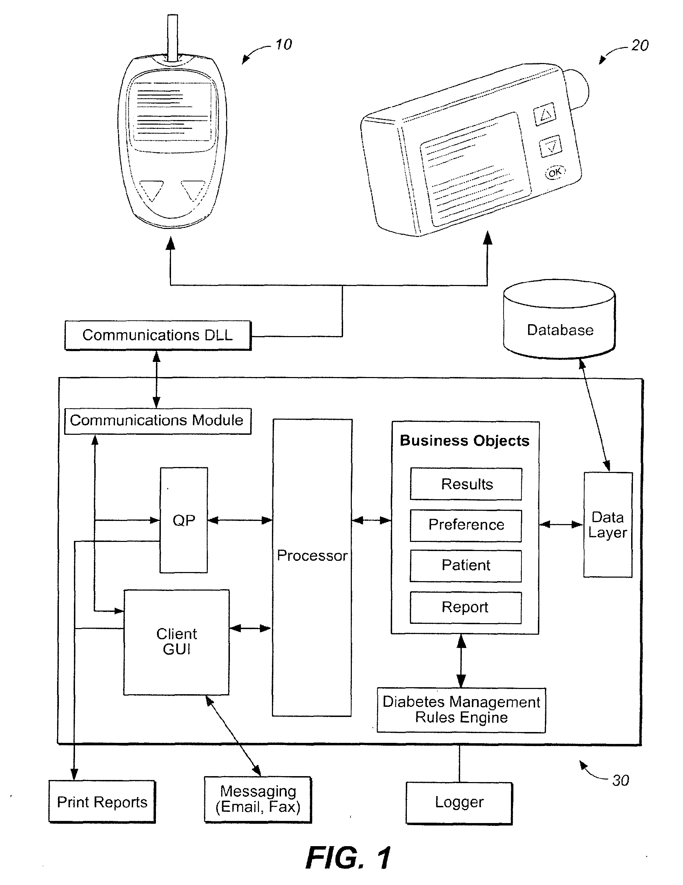

Figure 1 illustrates a schematic of a diabetes management system that includes a glucose meter 10, an insulin pump 20, and a personal computer (PC) 30. PC 30, illustrated in schematic form in Figure 1, may have a microprocessor unit and a memory unit. Glucose meter 10 may be configured to use a disposable test strip having a reagent such as, for example, glucose oxidase, ferricyanide, ruthamine hexamine, or combinations thereof. The reagent chemistry is capable of a physical transformation of glucose that allows a signal to be measured with glucose meter 10. In one embodiment, a diabetes management system or process may be installed on the memory unit in PC 30. In another embodiment, the diabetes management system or process may be installed on a memory unit of glucose meter 10, insulin pump 20, or other suitable computing device such as a personal digital assistant (PDA) or a cellular phone, i.e., any communication device with a processor and graphical user interface with a visual or audio output.

Glucose meter 10, insulin pump 20, and PC 30 may all have the ability to bi-directionally transfer data to each other. The data transfer process may be implemented in a wired or wireless manner. A cable may be used to transfer data in a wired manner through a suitable wire medium such as, for example, a universal serial bus (USB), serial port (RS232) or application specific connectors. The data transfer process may also use a suitable wireless medium with a wireless protocol such as, for example, infrared (IR), radio frequency (RF), WiFi (IEEE 802.11 wireless Ethernet standards), and Bluetooth or application specific wireless protocol.

Diabetes management system or process may include a communications dynamic link library (DLL), a communications module, a quick print module QP, a graphical user interface (GUI), business object module, a diabetes management rules engine, a data layer module, and a database module, as illustrated in Figure 1. Diabetes management system or process may be configured to generate reports, print reports, send reports via e-mail and fax, and to log errors via the logger, as illustrated in Figure 1.

The communications DLL may be an executable program module that allows PC 30 to recognize and communicate with glucose meter 10 and insulin pump 20. In addition, the communications DLL may allow PC 30 to communicate with several different types of glucose meters and insulin pumps and also a wide array of devices such as scales, sphygmomanometer, thermometers, pedometers, and heart rate monitors. The communication module may act as a surrogate by abstracting the lower level functionality that establishes connectivity with serial and USB devices

The quick print module QP may be a sub-routine configured to cause a glucose meter to seamlessly transfer data to PC 30 and then print a data report. After an initial setup, glucose meter 10 may be connected to PC 30 with a cable. Without having to manually launch a management application or perform any additional steps, the glucose meter will transfer its data and then print the data report. Details of the quick print module QP are shown and described in U.S. Patent Application S.N. 11/142,903 filed on May 31, 2005, which is incorporated by reference in its entirety herein.

The GUI may be a plurality of user interface screens that allow a user to configure and operate the diabetes management system or process. The screens can be configured as a touch screen or a combination of a display and a keyboard or buttons.

The business object module may be a central engine that will integrate and communicate with types of results, patient, preference, and reporting functionalities. The business object rules may be used by the GUI to generate results, reports, or other functionalities. As used herein, the term "patient" includes not only human subjects but also other mammals with indication of diabetes.

The data layer module may be an abstracted data access layer, which may act as an intermediate layer between the database module and the management application. The data layer module may execute the queries on the database module and return a record set, if applicable.

The database module may be a tool for storing and organizing data collected from glucose meter 10 and alternatively other devices. The database module may be installed, for example, on PC 30, a network server or transportable memory storage device.

The diabetes management rule engine may include a plurality of processes, devices, or sub-routines for analyzing data from glucose meter 10 and/or insulin pump 20. The plurality of sub-routines may apply statistical tests and triggers to analyze data so that messages can be provided to a user and/or a physician to warn about possible problem spots and/or compliance issues. A microprocessor may be configured to analyze data using the diabetes management rule engine. The diabetes management rule engine may be configurable by a physician and/or a user.

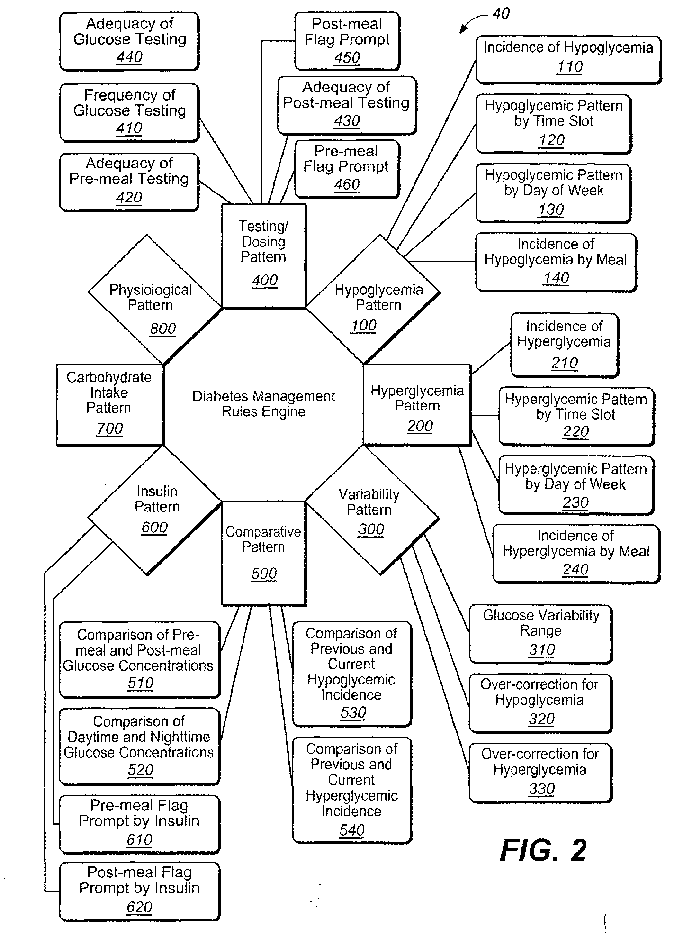

In one embodiment, the diabetes management rule engine may include a plurality of pattern recognition rules that can identify a testing/dosing pattern 400, a hypoglycemic pattern 100, a hyperglycemic pattern 200, a blood glucose variability pattern 300, a comparative pattern 500, and an insulin pattern 600, as illustrated in Figure 2. The testing/ dosing pattern 400 may include the following sub-routines, such as, for example, a frequency of glucose testing 410, an adequacy of pre-meal testing 420, an adequacy of post-meal testing 430, an adequacy of glucose testing 440, a post-meal flag prompt 450, and a pre-meal flag prompt 460. The hypoglycemic pattern 100 may include the following sub-routines such as, for example, an incidence of hypoglycemia 110, a hypoglycemic pattern by time slot 120, a hypoglycemic pattern by day of week 130, and an incidence of hypoglycemia by meal 140. The hyperglycemic pattern 200 may include the following sub-routines such as, for example, an incidence of hyperglycemia 210, a hyperglycemic pattern by time slot 220, a hyperglycemic pattern by day of week 230, and an incidence of hyperglycemia by meal 240. The blood glucose variability pattern 300 may include the following sub-routines such as, for example, a glucose variability range 310, an overcorrection for hypoglycemia 320, and an overcorrection for hyperglycemia 330. The comparative pattern 500 may include the following sub-routines such as, for example, a comparison of pre-meal and post-meal glucose concentrations 510, a comparison of daytime versus nighttime glucose concentrations 520, a comparison of previous and current hypoglycemic incidence 530, and a comparison of previous and current hyperglycemic incidence 540.

Alternatively, the plurality of pattern recognition rules may include an insulin pattern 600, a carbohydrate intake pattern 700 and a physiological pattern 800. The insulin pattern 600 may include the following sub-routines such as, for example, a pre-meal flag prompt by insulin 610 and a post-meal flag prompt by insulin 620.

The following will describe a description of the aforementioned pattern recognition rules (100, 200, 300, 400, 500, and 600).

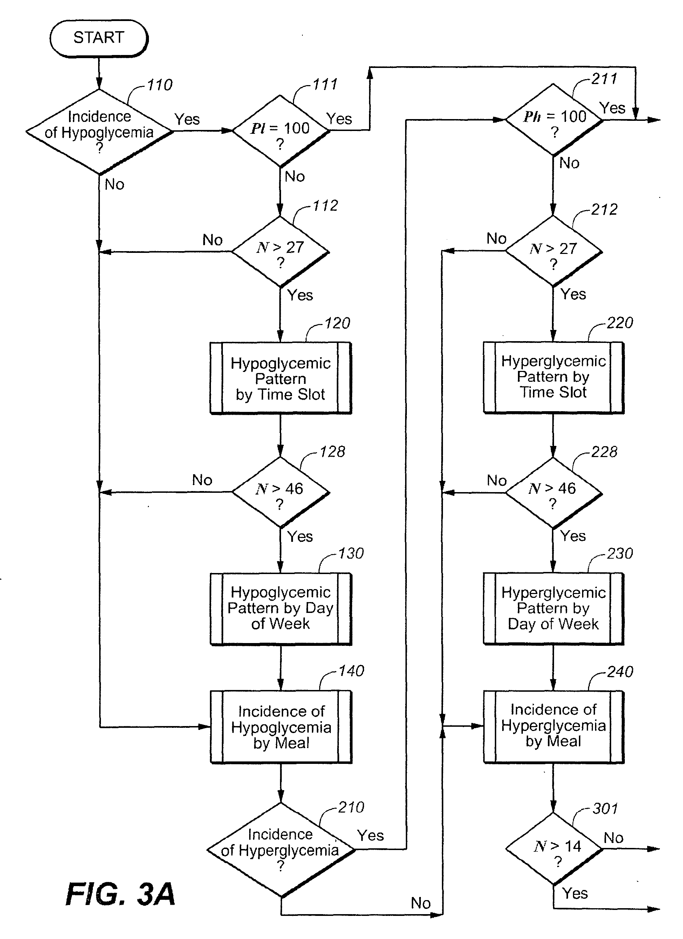

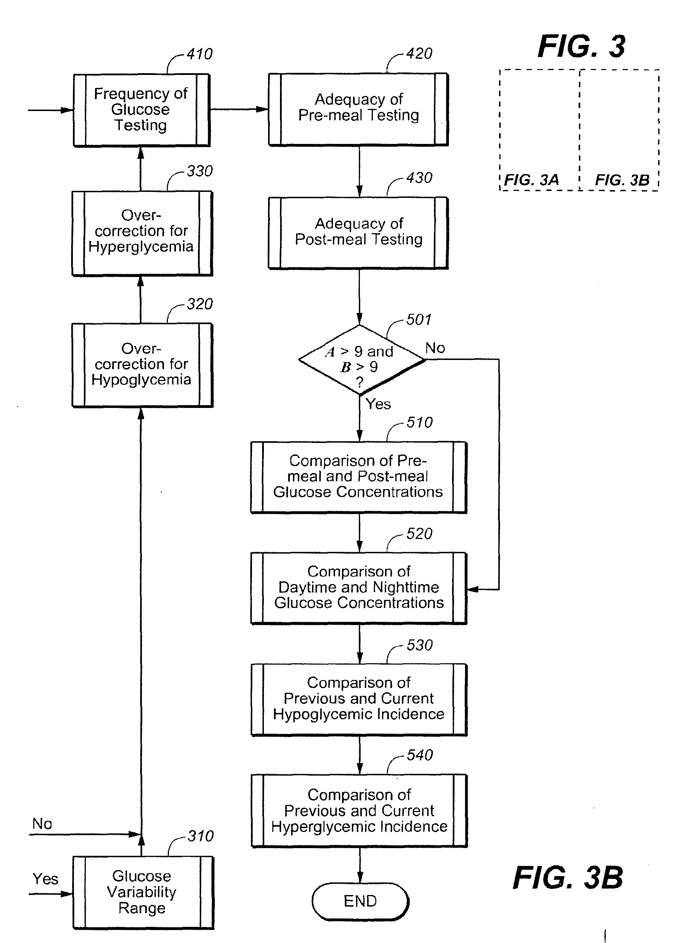

Figures 3A and 3B illustrate a flow chart of the diabetes management system or process system. A plurality of glucose concentration measurements may be processed using incidence of hypoglycemia sub-routine 110. If the percentage of hypoglycemic incidence $PI$ is greater than a pre-determined threshold, then the method moves to step 111 to determine whether the percentage of hypoglycemic incidence $PI$ equals about 100%. If the percentage of hypoglycemic incidence $PI$ in step 110 is less than a pre-determined threshold, then the method moves to the incidence of hypoglycemia by meal sub-routine 140. The pre-determined threshold for the incidence of hypoglycemia sub-routine 110 may range from about 3% to about 15%. In the preferred embodiment, the threshold is about 5%. Alternatively, the threshold may be of any value as selected by a clinician or physician. And as used herein, the term "about" or "approximately" in conjunction with a numerical value denotes that variations in the numerical value are intended as long as the variations allow the exemplary embodiments to perform for its intended purpose.

For step 111, if the hypoglycemic frequency $PI$ equals about 100%, then the method moves to the frequency of glucose testing sub-routine 410. However, if the hypoglycemic frequency $PI$ in step 111 does not equal about 100%, then the method moves to step 112 to determine whether more than about 27 glucose concentration measurements have been collected.

For step 112, if there are more than about 27 measurements, then the method moves to the hypoglycemic pattern by time slot sub-routine 120. However, if there are not more than about 27 measurements, then the method moves to the incidence of hypoglycemia by meal sub-routine 140. The sample size of 27 represents approximately the minimum number of glucose measurements required to perform sub-routine 120 and is based on several assumptions including: that a user tests at least once per day; that a user tests in at least two times per day; that there are at least two categories of observed incidences that are hypoglycemic incidences and not hypoglycemic incidences; and that the at least two categories each have greater than or equal to about five incidences. When using a chi-squared test, the minimum expected number of incidences is five for each category per time slot. Thus, two-time slots times two categories times five expected number of incidences gives approximately a minimum sample size of twenty. Based on the calculations, the sample size may be greater than about 20, and preferably be greater than about 27. For sample sizes greater than 20 such as, for example, 27 can be desirable based on a balancing test between consumer expectations of having the capability of receiving accurate warnings about their glycemic patterns without having to do an excessive number of glucose measurements, and having a sufficient number of glucose measurement to ensure a relative low number of false positive and false negative results.

The hypoglycemic pattern by time slot sub-routine 120 is performed if more than about 27 glucose concentration measurements were found in step 112. After performing the hypoglycemic pattern by time slot sub-routine 120, the method determines whether the plurality of glucose concentration measurements has more than about 46 measurements, as shown in step 128. If more than about 46 glucose concentration measurements have been collected, as shown in step 128, then the method moves to the hypoglycemic pattern by day of week sub-routine 130 and also to the incidence of hypoglycemia by meal sub-routine 140. If not more than about 46 glucose concentration measurements have been collected, as indicated in step 128, then the method moves to the incidence of hypoglycemia by meal sub-routine 140. The sample size of 46 measurements is based on several assumptions that include that a user tests at least once per day; that the user tests in at least five of the seven days per week; that there are at least two categories of observed incidences that are hypoglycemic incidences and not hypoglycemic incidences; and that the at least two categories each have greater than or equal to about five incidences for the at least five days of the week. When using a chi-squared test, the minimum expected number of incidences is five for each category per day of the week. Thus, five days time's two categories time's five expected counts give a minimum sample size of 50. Based on the calculations, the sample size may be greater than about 50, and preferably be greater than about 46. For sample sizes less than 50 such as, for example, 46 can be desirable based on a balancing test between consumer expectations of having the capability of receiving accurate warnings about their glycemic state without having to do an excessive number of glucose measurements, and having a sufficiently number of glucose measurements to ensure a relative low number of false positive and false negative results.

After analyzing the plurality of glucose concentration measurements using the pattern recognition rules of hypoglycemic pattern 100, the pattern recognition rules of hyperglycemic pattern 200 may be performed. Thus, after performing the incidence of hypoglycemia by meal sub-routine 140, the method moves to the incidence of hyperglycemia sub-routine 210. If the percentage of hyperglycemic incidence **Ph** is greater than a pre-determined threshold, then the method moves to step 211 to determine whether the percentage of hyperglycemic incidence **Ph** equals about 100%. If the percentage of hyperglycemic incidence **Ph** is less than a pre-determined threshold, then the method moves to the incidence of hyperglycemia by meal sub-routine 240. The pre-determined threshold for the incidence of hyperglycemia sub-routine 210 may range from about 15% to about 50%.

For step 211, if the hyperglycemic frequency Ph equals about 100%, then the method moves to the frequency of glucose testing sub-routine 410. However, if the hyperglycemic frequency Ph does not equal about 100%, then the method moves to step 212 to determine whether more than about 27 glucose concentration measurements have been collected.

For step 212, if there are more than about 27 measurements, then the method moves to the hyperglycemic pattern by time slot sub-routine 220. However, if there are not more than about 27 measurements, then the method moves to the incidence of hyperglycemia by meal sub-routine 240. The sample size of 27 represents approximately the minimum number of glucose measurements required to perform sub-routine 120 and is based on several assumptions including: that a user tests at least once per day; that a user tests in at least two times; that there are at least two categories of observed incidences that are hyperglycemic incidences and not hyperglycemic incidences; and that the at least two categories each have greater than or equal to about five incidences. When using a chi-squared test, the minimum expected number of incidences is five for each category per time slot. Thus, two-time slots times two categories times five expected number of incidences gives approximately a minimum sample size of twenty. Based on the calculations, the sample size may be greater than about 20, and preferably be greater than about 27. For sample sizes greater than 20 such as, for example, 27 can be desirable based on a balancing test between consumer expectations of having the capability of receiving accurate warnings about their glycemic patterns without having to do an excessive number of glucose measurements, and having a sufficient number of glucose measurement to ensure a relative low number of false positive and false negative results.

The hyperglycemic pattern by time slot sub-routine 220 is performed if more than about 27 glucose concentration measurements were found in step 212. After performing the hyperglycemic pattern by time slot sub-routine 220, the method determines whether the plurality of glucose concentration measurements has more than about 46 measurements, as shown in step 228. If more than about 46 glucose concentration measurements have been collected, as shown in step 228, then the method moves to the hyperglycemic pattern by day of week sub-routine 230 and also to the incidence of hyperglycemia by meal sub-routine 240. If not more than about 46 glucose concentration measurements have been collected, as indicated in step 228, then the method moves to an incidence of hyperglycemia by meal sub-routine 240. The sample size of 46 measurements is based on several assumptions including: that a user tests at least once per day; that the user tests in at least five of the seven days per week; that there are at least two categories of observed incidences that are hyperglycemic incidences and not hyperglycemic incidences; and that the at least two categories each have greater than or equal to about five incidences for the at least five days of the week. When using a chi-squared test, the minimum expected number of incidences is five for each category per day of the week. Thus, five days times two categories times five expected counts gives a minimum sample size of 50. Based on the calculations, the sample size may be greater than about 50, and preferably be greater than about 46. For sample sizes less than 50 such as, for example, 46 can be desirable based on a balancing test between consumer expectations of having the capability of receiving accurate warnings about their glycemic state without having to do an excessive number of glucose measurements, and having a sufficiently number of glucose measurements to ensure a relative low number of false positive and false negative results.

After analyzing the plurality of glucose concentration measurements using the pattern recognition rules of hyperglycemic pattern 200, the pattern recognition rules of variability pattern 300 may be performed. That is, after performing the incidence of hyperglycemia by meal sub-routine 240, the method moves to step 301 to determine whether more than about 14 glucose concentration measurements have been collected. If more than about 14 glucose concentration measurements have been collected, then the method moves to the glucose variability range sub-routine 310, the overcorrection for hypoglycemia sub-routine 320, and to the overcorrection for hyperglycemia sub-routine 330. If not more than about 14 glucose concentration measurements have been collected, then the method moves to the overcorrection for hypoglycemia sub-routine 320 and also to the overcorrection for hyperglycemia sub-routine 330. A sample size of 14 or greater may be selected to ensure the presence of a clinically significant pattern. Based on statistics, a sample size of 4 is sufficient to determine statistically significant difference, but a larger sample size was selected as a conservative measure to increase the likelihood of identifying a clinically significant pattern.

In addition to the methodologies described above, variability in blood glucose can be correlated, as will be described further herein, to a specific time period during a day, a plurality of time periods in a day, a specified day of a week, a plurality of specified days in a week, glucose testing frequency having pre-meal tests in a specific time period during a day, frequency of glucose measurements (i.e., testing) for pre-meal test for specified days of the week, glucose testing frequency having post-meal tests in a specific period during a day, frequency of glucose measurements (i.e.,

testing) for post-meal test for specified days of the week.

The pattern recognition rule of testing dosing pattern 400 may be performed after one of two condition are met, which are 1) the completion of the overcorrection for hyperglycemia sub-routine 330 or 2) the percentage of hyperglycemic incidence Ph or the percentage of hypoglycemic incidence *PI* equals about 100%, as illustrated in step 211 and step 111, respectively. If one of the two aforementioned conditions are met, then the method will then perform the following processes or sub-routines such as, for example, the frequency of glucose testing sub-routine 410, the adequacy of pre-meal testing sub-routine 420, and the adequacy of post-meal testing sub-routine 430.

After completing the pattern recognition rule of testing dosing pattern 400, the pattern recognition rules of comparative pattern 500 will be performed. As a first step in comparative pattern 500, the method will determine whether the number of blood glucose concentration measurements with a pre-meal flag A and the number of blood glucose concentration measurements with a post-meal flag B are both greater than about nine, as illustrated in step 501.

If both the number of blood glucose concentration measurements with a pre-meal flag A and the number of blood glucose concentration measurements with a post-meal flag *B* have more than about 9 flagged measurements, then the method will perform the comparison of pre-meal and post-meal glucose concentrations sub-routine 510, the comparison of daytime and nighttime glucose concentrations sub-routine 520, the comparison of previous and current hypoglycemic glucose incidence sub-routine 530, and the comparison of previous and current hyperglycemic incidence sub-routine 540. A sample size of 9 or greater may be selected to ensure the presence of a clinically significant pattern. Based on statistics, a sample size of 4 is sufficient to determine statistically significant difference, but a larger sample size was selected as a conservative measure to increase the likelihood of identifying a clinically significant pattern.

If either the number of blood glucose concentration measurements with a pre-meal flag *A* or the number of blood glucose concentration measurements with a post-meal flag *B* have less than about 9 flagged measurements, as illustrated in step 501, then the method will perform the comparison of daytime and nighttime glucose concentrations sub-routine 520, the comparison of previous and current hypoglycemic glucose incidence sub-routine 530, and the comparison of previous and current hyperglycemic incidence sub-routine 540.

The method may be completed after performing the comparison of previous and current hyperglycemic incidence sub-routine 540. The following will describe a more detailed description of the aforementioned processes or sub-routines (110, 120, 130, 140, 210, 220, 230, 240, 310, 320, 330, 410, 420, 430, 510, 520, 530, and 540).

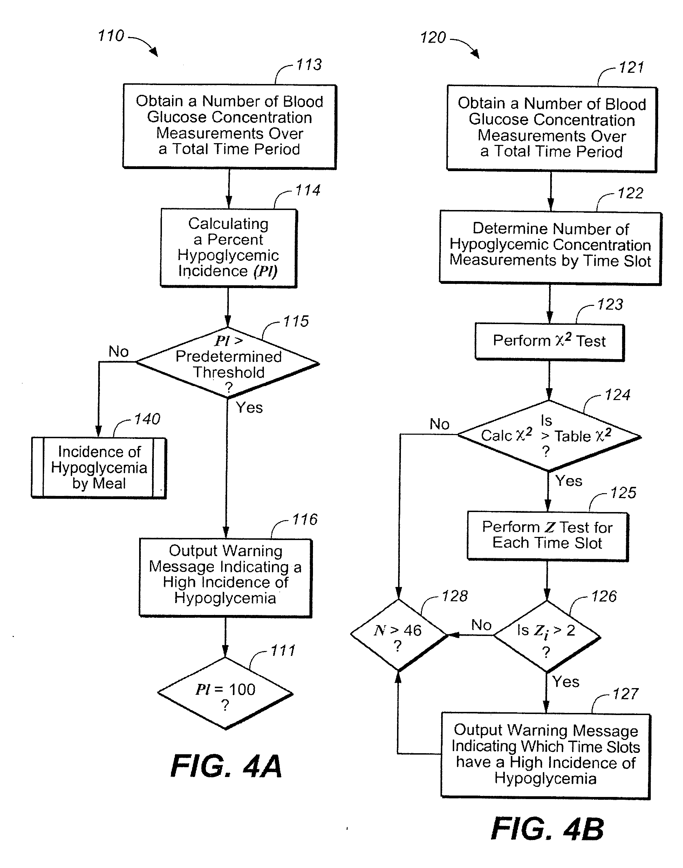

Figure 4A shows a flow chart of the incidence of hypoglycemia sub-routine 110, which may include obtaining a number of blood glucose concentration measurements over a total time period, as shown in step 113. Next, the sub-routine 110 may calculate a percentage of hypoglycemic incidence *PI* for a total time period by summing a number of substantially hypoglycemic blood glucose concentration measurements divided by a number of blood glucose concentration measurements collected over the total time period, as shown in step 114. The total time period can be arbitrarily selected time duration such as, for example, hours in a day, a day, a week, a month, three months, six months, or between visits to a physicians or therapeutic regimens. Equation 1 shows an example of how to calculate percentage of hypoglycemic incidence *PI*

$$Pl = \frac{\sum_{i=1}^{n} L_i}{\sum_{i=1}^{n} N_i} * 100 \qquad \text{Eq. 1}$$

In Equation 1, the term *i* represents a particular recurring time interval; *n* is a total number of recurring time intervals; and $L_i$ is a number of substantially hypoglycemic glucose concentration measurements that occur during time interval i; and $N_i$ represents the total number of glucose concentration measurements performed during time intervals i. The term $\sum_{i=1}^{n} L_i$ represents the total number of substantially hypoglycemic glucose concentration measurements for all of the recurring time intervals *i*. The term $\sum_{i=1}^{n} N_i$ represents the number of all glucose concentration measurements for all of the recurring time intervals *i*.

In step 115, the percentage of hypoglycemic incidence *PI* may be compared to a predetermined threshold. A message may be displayed indicating a high incidence of hypoglycemia if the percentage of hypoglycemic incidence *PI*

is greater than a predetermined threshold, as shown in step 116. If the percentage of hypoglycemic incidence *PI* is not greater than a pre-determined threshold, then the sub-routine 110 may move to the incidence of hypoglycemia by meal sub-routine 140. In one embodiment, the predetermined threshold may range from about 3% to about 15%. In the preferred embodiment, the threshold is about 5%. Alternatively, the threshold may be of any value as selected by a clinician or physician. After displaying a warning message in step 116, the sub-routine may move to step 111.

In particular, a hypoglycemic pattern by time slot sub-routine 120 may be used to determine if there is a high incidence of hypoglycemia occurring at a particular recurring time interval i. In one embodiment, the time interval may recur daily and be equal to about one eighth of a day. The eight daily time slots may include before breakfast, after breakfast, before lunch, after lunch, before dinner, after dinner, bedtime, and overnight, which can be pre-defined by default management settings or customized by the user. Note that a recurring time interval may also be referred to as a time slot.

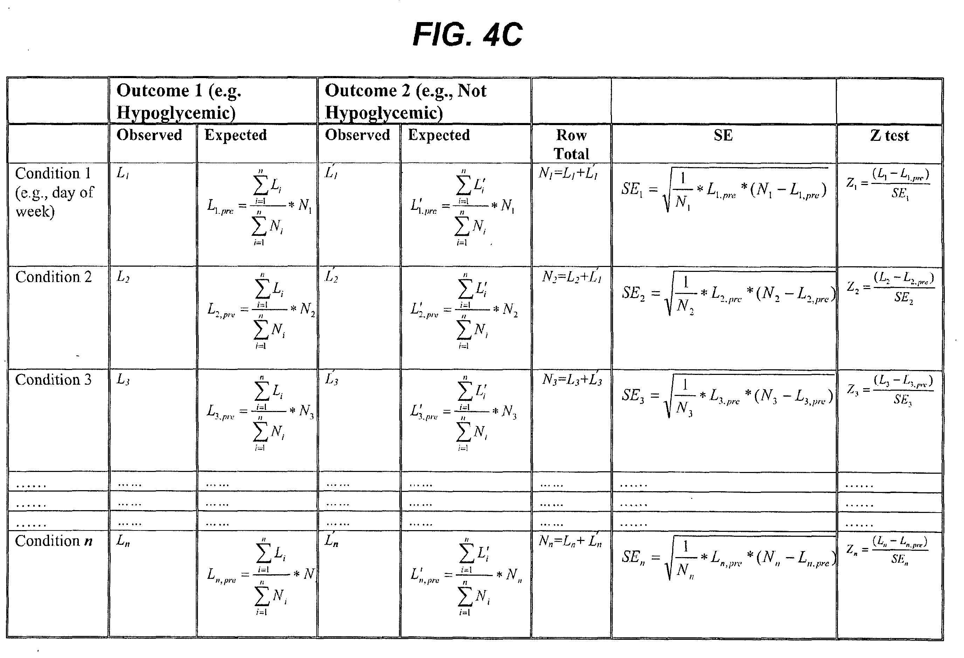

Figure 4B shows a flow chart of the hypoglycemic pattern by time slot sub-routine 120 that may include obtaining a number of blood glucose measurements over a total time period in step 121 and determining a number of hypoglycemic incidences for each of the time slots in step 122. Next, the sub-routine 120 determines whether the number of hypoglycemic incidence for at least one of the time slots is different using a statistical test such as, for example, a chi-squared test, and as shown in step 123. In step 124, the calculated chi-squared value is compared to a chi-squared value in a suitable table, shown here as a template in Figure 4C for a chi-squared table. It should be noted that, for brevity in the disclosure, the nomenclatures of the variables in this table in Figure 4C are the same nomenclatures provided in the forthcoming statistical analysis technique.

Referring back to Figure 4B, if the calculated chi-squared is not greater than a chi-squared value in a table (of which a template is shown in Figure 4C), then the sub-routine 120 moves to step 128. If the calculated chi-squared is greater than the chi-squared value in a table (of which a template is shown in Figure 4C), then the sub-routine120 moves to perform a Z test for each time slot, as shown in step 125. In one embodiment, the Z test may be a two-sided Z test. In step 126, the calculated $Z_i$ value is compared to a value of about 2. If the calculated $Z_i$ is greater than about 2, then a message indicating a high incidence of hypoglycemia has occurred at a particular time slot will be displayed, as shown in step 127. After displaying the message, the subroutine 120 moves to the step 128. If the calculated $Z_i$ is not greater than about 2, then the sub-routine 120 moves to the step 128.

In one embodiment, a chi-squared test may be used to determine if any of the time slots are statistically significantly different from each other. The chi-squared test may use a confidence level ranging from about 95% to about 99%. Equation 2 shows an example of how to calculate chi-squared $\chi^2$.

$$\chi^2 = \sum_{i=1}^{n} \frac{(L_i - L_{i,pre})^2}{L_{i.pre}} + \sum_{i=1}^{n} \frac{(L_i' - L_{i,pre}')^2}{L_{i.pre}'} \qquad \text{Eq. 2}$$

In Equation 2, the term $L'_i$ is a number of non-hypoglycemic glucose concentration measurements that occur during time interval *i*. $L_{i,pre}$ is a predicted number of substantially hypoglycemic glucose concentration measurements that will occur during time interval *i*. $L'_{i,pre}$ is a predicted number of non-hypoglycemic glucose concentration measurements that will occur during time interval *i*. After determining $\chi^2$ using Equation 2, the calculated $\chi^2$ value is compared to a $\chi^2$ in a table based on a number of degrees of freedom for each of the time intervals i. If the calculated $\chi^2$ is greater than the $\chi^2$ value on the table, then at least one of the time intervals is statistically significantly different.

The term $L_{i,pre}$ may be calculated using Equation 3a.

$$L_{i,pre} = \frac{\sum_{i=1}^{n} L_i}{\sum_{i=1}^{n} N_i} * N_i \qquad \text{Eq. 3a}$$

The term $L'_{i,pre}$ may be calculated using Equation 3b.

$$L'_{i,pre} = \frac{\sum_{i=1}^{n} L'_i}{\sum_{i=1}^{n} N_i} * N_i \qquad \text{Eq. 3b}$$

The term $\dfrac{\sum_{i=1}^{n} L_i}{\sum_{i=1}^{n} N_i}$ represents a fraction that estimates the likelihood of observing a hypoglycemic event based on all of the recurring time intervals combined.

The method of performing the hypoglycemic pattern by time slot sub-routine 120 may further include identifying which one of the recurring time intervals i is statistically significantly different using a Z test if the chi-squared test determines that at least one of the time intervals is statistically significantly different. Equation 4 shows an example of the Z *test.*

$$Z_i = \frac{(L_i - L_{i,pre})}{SE_i} \qquad \text{Eq. 4}$$

The term $Z_i$ represents a Z value at a particular time interval *i* and $SE_i$ represents a standard error for a particular time interval *i*. The term $SE_i$ may be calculated using Equation 5.

$$SE_i = \sqrt{\frac{1}{N_i} * L_{i,pre} * (N_i - L_{i,pre})} \qquad \text{Eq. 5}$$

A $Z_i$ value may be calculated for each recurring time interval i and compared to a **Z** value in a table. If the $Z_i$ value for one of the recurring time intervals is greater than the **Z** value in the table (e.g., about two), then the particular recurring time interval i is statistically significantly different.

The hypoglycemic pattern by day of the week sub-routine 130 may be performed in a manner similar to hypoglycemic pattern by time slot sub-routine 120. In the hypoglycemic pattern by day of the week sub-routine 130, the time intervals recur weekly where there are seven time slots to represent each day of the week.

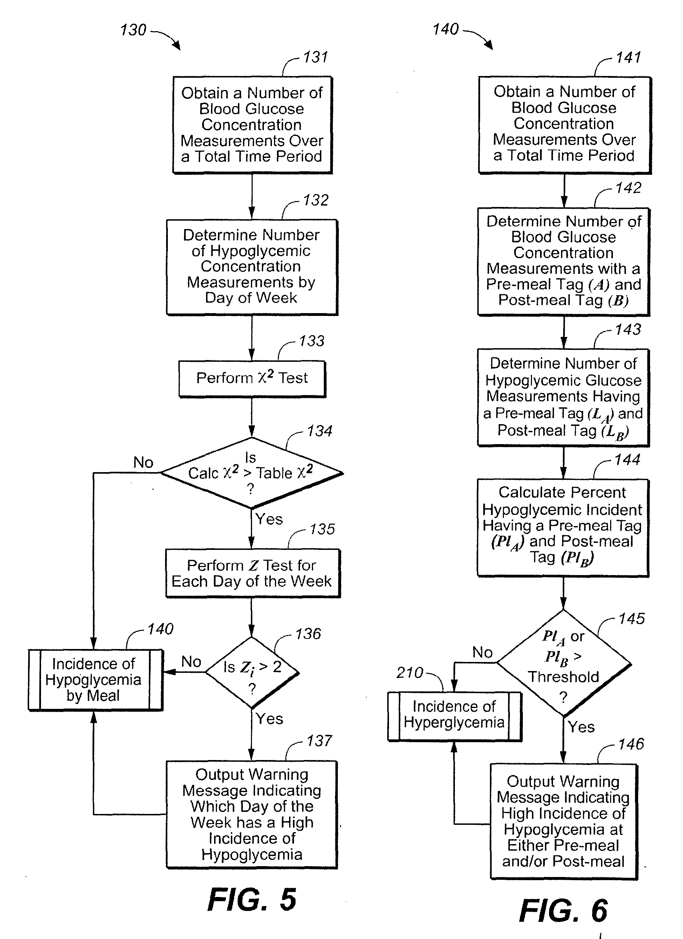

Figure 5 shows a flow chart of the hypoglycemic pattern by day of week 130 that may include obtaining a number of blood glucose measurements over a total time period in step 131 and determining a number of hypoglycemic incidences for each day in step 132. Next, the sub-routine 130 determines whether the number of hypoglycemic incidence for at least one of the days is different using a statistical test such as, for example, a chi-squared test (as shown in a template of Figure 4C), and as shown in step 133. The calculated chi-squared value is compared to a chi-squared value in a table, as shown in step 134. If the calculated chi-squared is not greater than a chi-squared value in a table (of which a template is shown in Figure 4C), then the sub-routine 130 moves to the next sub-routine140. If the calculated chi-squared is greater than the chi-squared value in a table (of which a template is shown in Figure 4C), then the sub-routine130 moves to perform a Z test for each day of the week, as shown in step 135. In step 136, the calculated $Z_i$ value is compared to a value of about 2. If the calculated $Z_i$ is greater than about 2, then a message indicating a high incidence of hypoglycemia has occurred at a particular day of the week will be displayed, as shown in step 137. After displaying the message, the subroutine 130 moves to the next sub-routine 140. If the calculated $Z_i$ is not greater than about 2, then the sub-routine 120 moves to the next sub-routine 140.

Figure 6 shows a flow chart of the hypoglycemia by meal sub-routine 140 that may be used to determine if there is a high incidence of hypoglycemia occurring at either a pre-meal or post-meal time interval. The incidence of hypoglycemia by meal sub-routine 140 may include obtaining a number of blood glucose concentration measurements over a total time period, as shown in step 141. A number of blood glucose concentration measurements with a pre-meal tag *A* and post-meal tag *B* may be calculated indicating the number of blood glucose measurement performed before eating a meal and after a meal, respectively, as shown in step 142. A number of substantially hypoglycemic blood glucose concentration measurements with a pre-meal tag $L_A$ and post-meal tag $L_B$ may be calculated, as shown in step 143. The percentage of hypoglycemic incidence having a pre-meal tag $PI_A$ and a post-meal tag $PI_B$ may be calculated, as indicated in step 144. $PI_A$ may be determined by dividing the number of substantially hypoglycemic blood glucose concentration measurements that have the pre-meal flag $L_A$ by the number of blood glucose concentration measurements with the pre-meal tag A. Similarly, $PI_B$ may be determined by dividing the number of substantially hypoglycemic blood glucose concentration measurements that have the pre-meal flag $L_B$ by the number of blood glucose concentration measurements with a pre-meal tag *B.*

Equations 6 and 7 illustrate a mathematical embodiment on how to determine the percentage of hypoglycemic incidence having a pre-meal tag $PI_A$ and a post-meal tag $PI_B$.

$$Pl_A = \frac{L_A}{A} * 100 \qquad \text{Eq. 6}$$

$$Pl_B = \frac{L_B}{B} * 100 \qquad \text{Eq. 7}$$

The percentage of hypoglycemic incidence having a pre-meal tag $PI_A$ and a post-meal tag $PI_B$ may be compared to a pre-determined threshold, as shown in step 145. If either $PI_A$ or $PI_B$ is greater than a pre-determined threshold, then a message can be displayed indicating a high incidence of hypoglycemia occurring at a pre-meal time and/or a post-meal time, as shown in step 146. If $PI_A$ and $PI_B$ are not greater than a predetermined threshold, then sub-routine 140 may move to the incidence of hyperglycemia sub-routine 210. After displaying a message in step 146, the sub-routine 140 may move to the incidence of hyperglycemia sub-routine 210. In one embodiment, the pre-determined threshold may range from about 10% to about 25%.

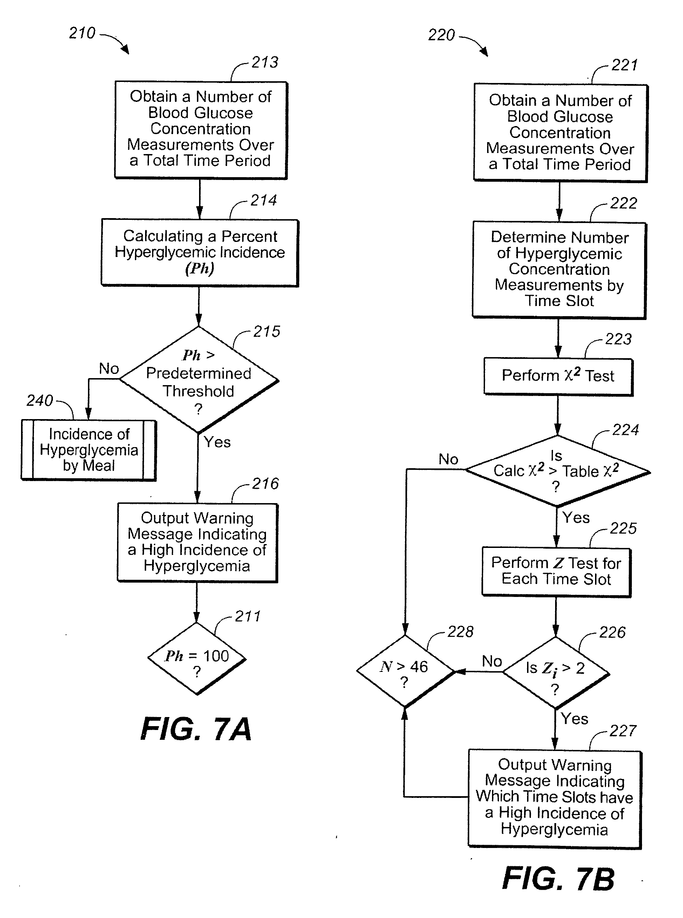

Figure 7A shows a flow chart of the incidence of hyperglycemia sub-routine 210, which may include obtaining a number of blood glucose concentration measurements over a total time period, as shown in step 213. The total time period can be any arbitrarily selected time duration, such as, for example, number of hours in a day, one day, one week, one month, three months, six months, time between visits to a physician's office, and so on. Next, the sub-routine 210, as implemented in a suitable computer, may calculate a percentage of hyperglycemic incidence *Ph* for a total time period by summing a number of substantially hyperglycemic blood glucose concentration measurements divided by a number of blood glucose concentration measurements collected over the total time period, as shown in step 214. Equation 8 shows an example of how to calculate percentage of hyperglycemic incidence *Ph*

$$Ph = \frac{\sum_{i=1}^{n} H_i}{\sum_{i=1}^{n} N_i} * 100 \qquad \text{Eq. 8}$$

The term *i* represents a particular recurring time interval; *n* is a total number of recurring time intervals; and $H_i$ is a number of substantially hyperglycemic glucose concentration measurements that occur during time interval *i*; and $N_i$ represents the total number of glucose concentration measurements performed during time intervals *i*. Then term

$\sum_{i=1}^{n} H_i$ represents the total number of substantially hyperglycemic glucose concentration measurements for all of the recurring time intervals *i*. The term $\sum_{i=1}^{n} N_i$ represents the number of all glucose concentration measurements for all of the recurring time intervals *i*.

In step 215, the percentage of hyperglycemic incidence **Ph** may be compared to a predetermined threshold. A message may be displayed indicating a high incidence of hyperglycemia if the percentage of hyperglycemic incidence Ph is greater than a pre-determined threshold, as shown in step 216. If the percentage of hyperglycemic incidence **Ph** is not greater than a pre-determined threshold, then the sub-routine 110 may move to the incidence of hyperglycemia by meal sub-routine 240. In one embodiment, the pre-determined threshold may range from about 15% to about 50%. After displaying a warning message in step 216, the sub-routine may move to step 211.

The hyperglycemic pattern by time slot sub-routine 220 may be used to determine if there is a high incidence of hyperglycemia occurring at a particular recurring time interval i. Figure 7A shows a flow chart of the hyperglycemic pattern by time slot sub-routine 220 that may include obtaining a number of blood glucose measurements over a total time period in step 221 and determining a number of hyperglycemic incidences for each of the time slots in step 222. Next, the sub-routine 220 determines whether the number of hyperglycemic incidence for at least one of the time slots is different using a statistical test such as, for example, a chi-squared test (as shown in a template of Figure 4C), and as shown in step 223. The calculated chi-squared value is compared to a chi-squared value in a table, as shown in step 224. If the calculated chi-squared is not greater than a chi-squared value in a table (of which a template is shown in Figure 4C), then the sub-routine 220 moves to step 228. If the calculated chi-squared is greater than the chi-squared value in a table (of which a template is shown in Figure 4C), then the sub-routine 220 moves to perform a Z test for each time slot, as shown in step 225. In one embodiment, the Z test may be a two-sided Z test. In step 226, the calculated $Z_i$ value is compared to a value of about 2. If the calculated $Z_i$ is greater than about 2, then a message indicating a high incidence of hyperglycemia has occurred at a particular time slot will be displayed, as shown in step 227. After displaying the message, the subroutine 220 moves to the step 228. If the calculated $Z_i$ is not greater than about 2, then the sub-routine 220 moves to the step 228.

In one embodiment, a chi-squared test may be used to determine if any of the time slots are statistically significantly different. The chi-squared test may use a confidence level ranging from about 95% to about 99%. Equation 9 shows an example of how to calculate chi-squared $\chi^2$.

$$\chi^2 = \sum_{i=1}^{n} \frac{(H_i - H_{i,pre})^2}{H_{i,pre}} + \sum_{i=1}^{n} \frac{(H_i' - H_{i,pre}')^2}{H_{i,pre}'} \qquad \text{Eq. 9}$$

In Equation 9, the term $H_i'$ is a number of non-hyperglycemic glucose concentration measurements that occur during time interval *i*. $H_{i,pre}$ is a predicted number of substantially hyperglycemic glucose concentration measurements that will occur during time interval *i*. $H'_{i,pre}$ is a predicted number of non-hyperglycemic glucose concentration measurements that will occur during time interval *i*. After determining $\chi^2$ using Equation 9, the calculated $\chi^2$ value is compared to a $\chi^2$ in a table based on a number of degrees of freedom for each of the time intervals i. If the calculated $\chi^2$ is greater than the $\chi^2$ value on the table, then at least one of the time intervals is statistically significantly different.

The term $H_{i,pre}$ may be calculated using Equation 10a.

$$H_{i,pre} = \frac{\sum_{i=1}^{n} H_i}{\sum_{i=1}^{n} N_i} * N_i \qquad \text{Eq. 10a}$$

The term $H'_{i,pre}$ may be calculated using Equation 10b.

$$H'_{i,pre} = \frac{\sum_{i=1}^{n} H'_i}{\sum_{i=1}^{n} N_i} * N_i \qquad \text{Eq. 10b}$$

The term $\dfrac{\sum_{i=1}^{n} H_i}{\sum_{i=1}^{n} N_i}$ represents a fraction that estimates the likelihood of observing a hyperglycemic event based on all of the recurring time intervals combined.

The method of performing the hyperglycemic pattern by time slot sub-routine 220 in Figure 7B may further include identifying which one of the recurring time intervals $i$ is statistically significantly different using a *Z* test if the chi-squared test determines that at least one of the time intervals is statistically significantly different. Equation 11 shows an example of the *Z test.*

$$Z_i = \frac{(H_i - H_{i,pre})}{SE_i} \qquad \text{Eq. 11}$$

In Equation 11, the term $Z_i$ represents a *Z* value at a particular time interval i and $SE_i$ represents a standard error for a particular time interval *i*. The term $SE_i$ may be calculated using Equation 12.

$$SE_i = \sqrt{\frac{1}{N_i} * H_{i,pre} * (N_i - H_{i,pre})} \qquad \text{Eq. 12}$$

A $Z_i$ value may be calculated for each recurring time interval i and compared to a *Z* value in a table. If the $Z_i$ value for one of the recurring time intervals is greater than the *Z* value in the table (e.g., about two), then the particular recurring time interval *i* is statistically significantly different.

The hyperglycemic pattern by day of the week sub-routine 230 may be performed in a manner similar to hyperglycemic pattern by time slot sub-routine 220. In the hyperglycemic pattern by day of the week sub-routine 230, the time intervals recur weekly where there are seven time slots to represent each day of the week.

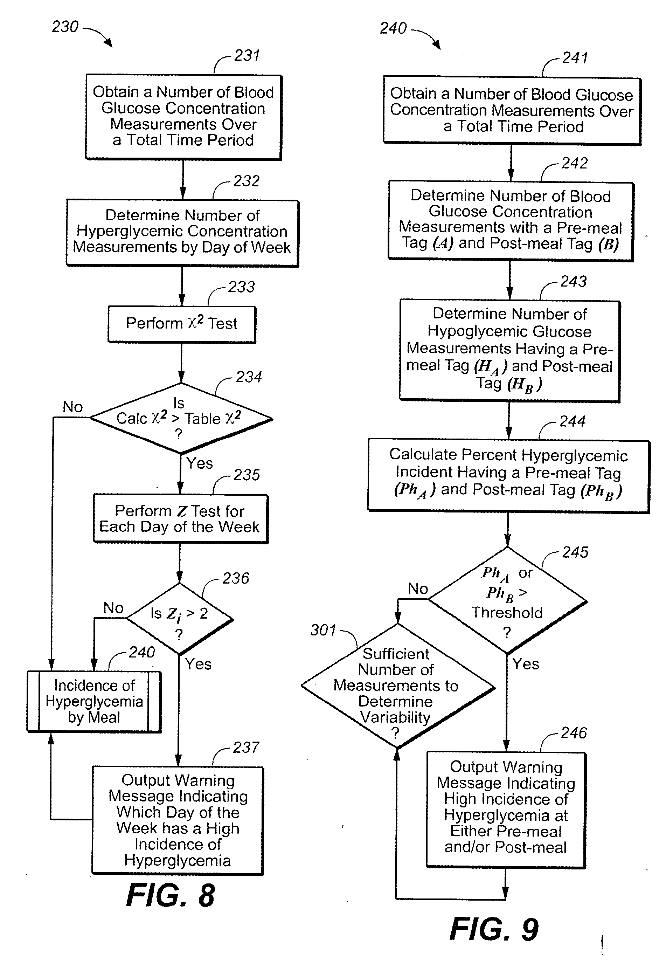

Figure 8 shows a flow chart of the hyperglycemic pattern by day of week 230 that may include obtaining a number of blood glucose measurements over a total time period in step 231 and determining a number of hyperglycemic incidences for each day in step 232. Next, the sub-routine 230, as implemented on a suitable computing device, determines whether the number of hyperglycemic incidence for at least one of the days is different using a statistical test such as, for example, a chi-squared test (as shown in a template of Figure 4C), and as shown in step 233. The calculated chi-squared value is compared to a chi-squared value in a table, as shown in step 234. If the calculated chi-squared is not greater than a chi-squared value in a table (of which a template is shown in Figure 4C), then the sub-routine 230 moves to the next sub-routine 240. If the calculated chi-squared is greater than the chi-squared value in a table (of which a template is shown in Figure 4C), then the sub-routine 230 moves to perform a *Z* test for each day of the week, as shown in step 235. In step 236, the calculated $Z_i$ value is compared to a value of about 2. If the calculated $Z_i$ is greater than about 2, then a message indicating a high incidence of hyperglycemia has occurred at a particular day of the week will be displayed, as shown in step 237. After displaying the message, the subroutine 230 moves to the next sub-routine 240. If the calculated $Z_i$ is not greater than about 2, then the sub-routine 220 moves to the next sub-routine 240.

Figure 9 shows a flow chart of the hyperglycemia by meal sub-routine 240 that may be used to determine if there is a high incidence of hyperglycemia occurring at either a pre-meal or post-meal time interval. The incidence of hyperglycemia by meal sub-routine 240 may include obtaining a number of blood glucose concentration measurements over a total time period, as shown in step 241. A number of blood glucose concentration measurements with a pre-meal tag **A** and post-meal tag **B** may be calculated indicating the number of blood glucose measurement performed before eating a meal and after a meal, respectively, as shown in step 242. A number of substantially hyperglycemic blood glucose concentration measurements with a pre-meal tag $H_A$ and post-meal tag $H_B$ may be calculated, as shown in step 243. The percentage of hyperglycemic incidence having a pre-meal tag $Ph_A$ and a post-meal tag $Ph_B$ may be calculated, as indicated in step 244. $Ph_A$ may be determined by dividing the number of substantially hyperglycemic blood glucose concentration measurements that have the pre-meal flag $H_A$ by the number of blood glucose concentration measurements with the pre-meal tag **A**. Similarly, $Ph_B$ may be determined by dividing the number of substantially hyperglycemic blood glucose concentration measurements that have the pre-meal flag $H_B$ by the number of blood glucose concentration measurements with a pre-meal tag **B**.

Equations 13 and 14 illustrate a mathematical embodiment on how to determine the percentage of hyperglycemic incidence having a pre-meal tag $Ph_A$ and a post-meal tag $Ph_B$.

$$Ph_A = \frac{H_A}{A} * 100 \qquad \text{Eq. 13}$$

$$Ph_B = \frac{H_B}{B} * 100 \qquad \text{Eq. 14}$$

The percentage of hyperglycemic incidence having a pre-meal tag $Ph_A$ and a post-meal tag $Ph_B$ may be compared to a pre-determined threshold, as shown in step 245. If either $Ph_A$ or $Ph_B$ is greater than a pre-determined threshold, then a message can be displayed indicating a high incidence of hyperglycemia occurring at a pre-meal time and/or a post-meal time, as shown in step 246. If $Ph_A$ and $Ph_B$ are not greater than a pre-determined threshold, then sub-routine 240 may move to step 301, which is a first step in variability pattern rules 300. After displaying the message in step 246, the sub-routine 240 may move to the step 301. In one embodiment, the pre-determined threshold may range from about 15% to about 50%.

Referring to Figures 4-8, a method is provided that includes storing a patient's data that includes blood glucose concentration measurements; generating from the patient's data a suitable table having predetermined conditions (e.g., Time or Day) and outcomes (e.g., Hypoglycemic, Hyperglycemic or Other condition) upon indication of instances of hypoglycemia, hyperglycemia, or excessive blood glucose variability by time of day, by day in a week, both by time of day and day of week, or at different time intervals; calculating standard error (SE) and Z test with data from the table; and displaying a message when the Z test being greater than a predetermined value indicative of a pattern of glycemia outside at least a predetermined range for such pattern. In the preferred embodiment, a threshold for the Z test is about 2.

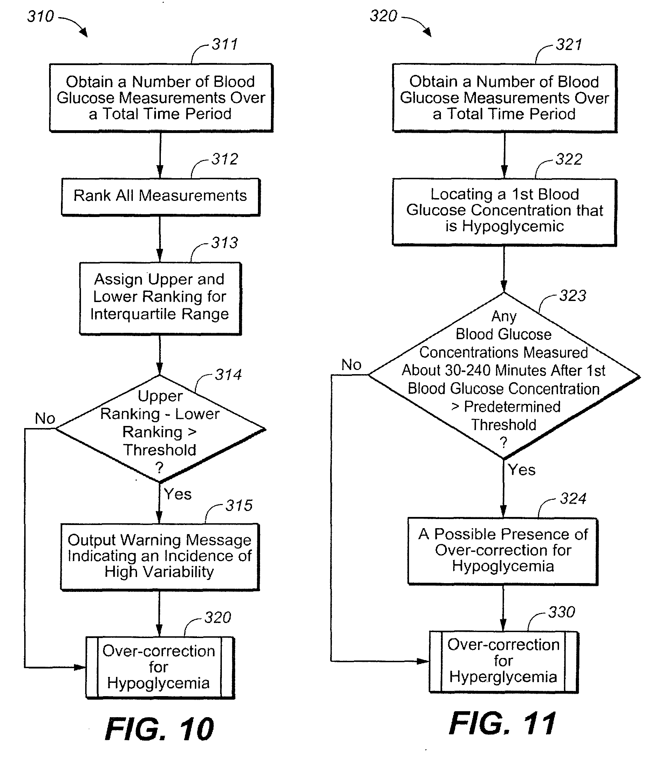

The glucose variability range sub-routine 310 may be used to indicate to a user if their glucose concentration has a wide range of blood glucose variability, as illustrated in Figure 10. The glucose variability range sub-routine 310 may include obtaining a number of blood glucose measurements over a total time period in step 311, and ranking all of the blood glucose measurements based on a magnitude of the blood glucose measurement in step 312. Next, an inter quartile range may be determined that includes an upper ranking and a lower ranking in step 313. The upper ranking may correlate to an upper glucose concentration and the lower ranking may correlate to a lower glucose concentration. The inter quartile range selected here can be a glucose measurement in the 75th and 25th percentile. However, other suitable ranges can also be utilized, such as, for example, 80th and 20th percentiles or 90th and 10th percentiles. In step 314, the upper glucose concentration is subtracted from the lower glucose concentration to calculate a differential value or, for example, an inter quartile range. If the differential value is greater than a pre-determined threshold, a message may be displayed indicating an incidence of high blood glucose variability as shown in step 315. If the differential value is not greater than a pre-determined threshold, the sub-routine 310 may move to the overcorrection for hypoglycemia sub-routine 320. After displaying the message in step 315, the sub-routine 310 may move to the overcorrection for hypoglycemia sub-routine 320. In one embodiment, the message is displayed only if there is a statistically significant number of blood glucose measurements collected by the glucose meter such as, for example, about greater than about fourteen blood glucose measurements, as shown in step 301 in Figure 3A. An example of the pre-determined threshold for the glucose variability range sub-routine 310 may range from about 30 mg/dL to about 90 mg/dL, and preferably about 50 mg/dL.

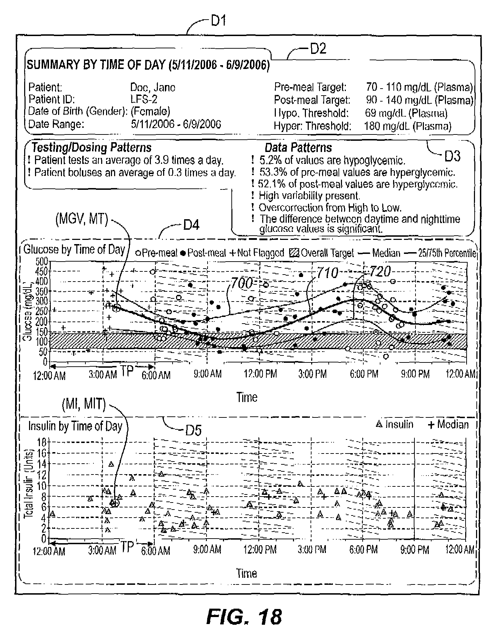

Variability can also be associated graphically with incidences of hypoglycemia or hyperglycemia at a specified time of day or in association with a meal slot. Other associations of blood glucose variability can be with a specified date, day of week, timing of meals or insulin injections. Specifically, the system performs a generating of blood glucose variability pattern by determining (a) a median of glucose concentration values during a temporal period and (b) a median of test times during the temporal period; and correlating (a) the median of glucose concentration values and (b) the median of test times to define a data point on a two-dimensional coordinate graph having glucose values and test times. A suitable range (e.g., interquartile, 10th and 90th percentiles or 20th and 80th percentiles) can be plotted around each median data point. As shown in an example in Figure 18, the temporal time period is selected to be a time period TP from 3:00 AM to 8:00 AM where a median of glucose concentration values is indicated by MGV of approximately 325 mg/dL during this time period with a median of the number of test measurements MT being approximately 4:00 AM, and both MGV and MT can be utilized to define a median data point on a two-dimensional chart for glucose value and time in display area D4. Association of blood glucose variability for MGV and MT can be determined for other specified indicators such as, for example, pre or post meal glucose concentration levels by time slot in a day or by days in a week or month, hypoglycemia, hyperglycemia, day of week, dates of week, or any time related specified indicator as deemed suitable by the user, patient, physician, or clinician. Thereafter, a suitable variability indicator (such as, for example, the interquartile range defined as a difference between the 75th and 25th percentile) can be determined around each of the median data. In the example illustrated in Figure 18, the data values defining the 75th percentile can be connected together as smoothed curve 700 about the median values defining curve 710 with the 25th percentile values defining curve 720. The curves 700 and 720 serve to show graphically the blood glucose variability around the median value associated with a specified indicator (e.g., glucose, insulin, or other physiological indicators).

In the preferred embodiments, the median is a preferred indicator of a tendency in the blood glucose data to centralize about some value, i.e., a central tendency. The median is also preferred over other indicator such as, for example, the arithmetic mean because it has been observed that measurement data from blood glucose meter do not follow a normal or *Gaussian* distribution (i.e., an asymmetric instead of symmetric distribution), as it would be for other indicators. Further, the use of the median is preferred because (a) the median is insensitive to outlier data, and (b) the median is essentially unaffected by values outside a range of measurement of blood glucose meters. It is believed values for the median are highly correlated to the mean, and that correlation between median blood glucose level and HbA1c values would be very close to the mean blood glucose and HbA1c. However, for precision, it is believed that more sampling data would be required the median as compared to the mean.

Referring back to Figure 18, the respective curves 700, 710, and 720 can be generated by a suitable interpolation technique, i.e., "curve smoothing," such as, for example, polynominal interpolation, cubic-Bezier spline, cubic cardinal spline, Fritsch-Carlson monotony preserving cubic interpolation, Akima cubic spline interpolation, rational cubic spline, or exponential interpolation. In the preferred embodiments, the curve smoothing is generated via a cardinal spline through a specified array of point structures using a tension of 1.

Heretofore, a method of assessing glycemia of a patient can be provided to provide a graphical comparison of insulin intake and blood glucose along with any other physiological parameters. The method can be achieved by collecting data related to a patient glycemia, including blood glucose measurements and insulin intake values; determining a combined median of glucose and time as a function of a median of blood glucose values and a median of time periods for each measurement of the blood glucose values over a predetermined temporal period; and displaying the combined median of glucose and time in a graphical format. The collecting can include collecting data of the patient over a plurality of temporal time periods. The determining can include determining the combined median of glucose and time for each of the plurality of temporal time periods. In particular, the displaying can include generating a graphical chart for each of the determining and establishing, where the charts have substantially the same temporal time periods.

Once the combined median of glucose and time has been determined, blood glucose variability can be generated by a suitable technique, such as, for example, using the inter quartile range. To show trends or patterns, the blood glucose variability can be obtained for each combined median of glucose and time over the plurality of temporal periods. And as used herein, the temporal periods can be any unit indicator of time such as for example, every 4 hours, every 8 hours, every 24 hours, day or days in a week, specific dates, every week or every month and so on.

The system can be utilized to associate the variability of glucose concentration with the intake of insulin via a common specified indicator (e.g., time of day, day of week, and others) to assess the effects of insulin, types of insulin, or frequency of insulin intake. Specifically, the system performs a generating of blood glucose variability pattern by determining (based on a common indicator of temporal time TP) the following: (i) a median of insulin doses MI taken by the patient during the temporal period selected above and (ii) a median of dosage times MIT during the temporal period and it would correlate both (i) the median of insulin doses MI and (ii) the median of the number of insulin intake values MIT to define a data point on a two-dimensional coordinate graph having insulin doses and dosage times as its ordinate and abscissa, respectively. In particular, the method above can be implemented to establish a combined median of insulin intake and time as a function of both (i) median of insulin intake and (ii) a median of time periods for each insulin intake over the predetermined temporal period; and displaying the combined median of insulin intake and time in a graphical format so that a clinician, patient or diabetes specialist would be able to assess generally the effect of insulin intake and blood glucose. This correlation of the median insulin doses and dosage time can be plotted graphically in an exemplary two-dimensional chart within display area D5, which then can be utilized to show the association in the variability of glucose in the chart of display area D4 and insulin in the chart of display area D5 by the common specified indicator of "time of day." It should be noted that the chart in display area D4 or D5 is not limited to a two-dimensional chart but that other types of charts can be utilized such as, for example, three-dimensional charts or charts using graphical representation for more than 4 different variable data inputs.

Although blood glucose variability has been described generally in relation to the median and interquartile range of blood glucose values, other techniques can be utilized, such as, for example:

"standard deviation" or SD,

"coefficient of variation" CV,

"average-daily-blood-glucose",

"N70+N180" where the number of blood glucose below 70 mg/dL and the number above 180 mg/dL are utilized,

"M value" derived as a composite measure of glycemic control from blood glucose data, as described by J. Schlichtkrull et al., The M-Value, an Index of Blood-Sugar Control in Diabetics, Acta Medica Scandinavia, Vol. 177, fasc. 1, 1965, pp. 95-93,

"Mean-Amplitude-Of-Glycemic-Excursion," as discussed by F. John Service et al., in Mean Amplitude of Glycemic Excursions, a Measure of Diabetic Instability, Diabetes, Vol. 19, No. 9, pp. 644-655, September 1970,

"Lability Index" as described by Kovatchev BP et al., in Methods for Quantifying Self-monitoring Blood Glucose Profile Exemplified by an Examination of Blood Glucose Pattern in Patients with Type I and Type 2 Diabetes, Diabetes Technology and Therapeutics, 4: 295-303, 2002,

"Absolute-Blood-Glucose-Rate-Of-Change" for readings less than 4 hours apart as discussed by Ryan EA et al., in Assessment of the Severity of Hypoglycemia and Glycemic Lability in Type 1 Diabetic Subjects Undergoing Islet Transplantation, Diabetes 53: 955-962, 2004,

"Figure of Merit," as described by Rodbard, D. (2005), Improved Methods for Calculating a "Figure of Merit" for Blood Glucose Monitoring Data, Diabetes Technology Meeting, San Francisco, CA, November 2005.

"J-index," as described by Wojcicki, J. (1995), J-Index, A New Proposition Of The Assessment Of Current Glucose Control In Diabetic Patients, Horm Metab Res., 27, 41-42, and

"Average-Daily-Risk-Range" as described by Otto et al., in Diabetes Care, Vol. 29, No. 11, pp. 2433-2438 (November 2006).

Other documents relating to the potential hazards posed by variability in blood glucose values are described in Hirsch IB. Glycemic Variability: It's Not Just About A1C Anymore! Diabetes Technol Ther. 2005;7:780-783; Brownlee M, Hirsch, I.B. Glycemic variability: A Hemoglobin A1c - Independent Risk Factor For Diabetic Complications. JAMA 2006;295(14): 1707-1708; and Monnier L, Mas E, Ginet C, et al., Activation Of Oxidative Stress By Acute Glucose Fluctuations Compared With Sustained Chronic Hyperglycemia In Patients With Type 2 Diabetes. JAMA. 2006;295: 1681-1687. The above-cited documents are hereby incorporated by reference in their entireties into this application.

The overcorrection for hypoglycemia sub-routine 320, as illustrated in Figure 11, may be used to determine if a user has ingested a bolus of carbohydrate that caused the user's blood glucose concentration to increase from a hypoglycemic state to a hyperglycemic state. Ideally, a user would want to ingest a bolus of carbohydrate to cause a switch from the hypoglycemic state to the euglycemic state.

The overcorrection for hypoglycemia sub-routine 320 may include obtaining a number of blood glucose measurements over a total time period as shown in step 321, and measuring a first blood glucose concentration that is less than a first pre-determined threshold, as shown in step 322. The first pre-determined threshold may be about 70 mg/dL where a blood glucose concentration that is less than the first pre-determined threshold is hypoglycemic. The first blood glucose concentration indicates that the user is in a hypoglycemic state. In step 323, all blood glucose measurements performed from about 30 minutes to about 240 minutes after the first blood glucose concentration measurement are evaluated for hyperglycemia. If one of the blood glucose concentrations are found to be greater than about a second pre-determined threshold, then a message is displayed indicating a possible presence of overcorrection for hypoglycemia, as shown in step 324. The second pre-determined threshold may be about 180 mg/dL. If none of the blood glucose concentrations are found to be greater than about the second pre-determined threshold, then the subroutine 320 may move to the overcorrection for hypoglycemia sub-routine 330. After displaying the message in step 324, the sub-routine 320 may move to the overcorrection for hyperglycemia sub-routine 330.

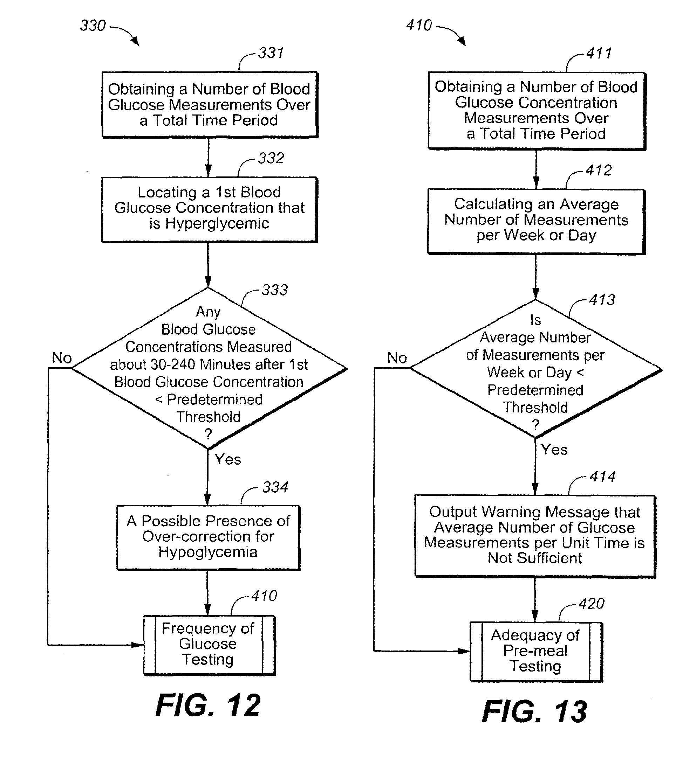

The overcorrection for hyperglycemia sub-routine 330, as illustrated in Figure 12, may be used to determine if a user has taken a bolus of insulin such that the user's glucose concentration decreased from a hyperglycemic state to a hypoglycemic state. Ideally, a user may want an insulin bolus to cause a switch from the hyperglycemic state to the euglycemic state.

The overcorrection for hyperglycemia sub-routine 330 may include obtaining a number of blood glucose measurements over a total time period as shown in step 331, and measuring a first blood glucose concentration that is greater than a second pre-determined threshold, as shown in step 332. The second pre-determined threshold may be about 180 mg/dL where a blood glucose concentration that is greater than the second pre-determined threshold is hyperglycemic. In step 333, all blood glucose measurements performed from about 30 minutes to about 240 minutes after the first blood glucose concentration measurement are evaluated for hypoglycemia. If one of the blood glucose concentrations are found to be less than about a first pre-determined threshold, then a message is displayed indicating a possible presence of overcorrection for hyperglycemia, as shown in step 334. The first pre-determined threshold may be about 70 mg/dL. If none of the blood glucose concentrations are found to be less than about the first pre-determined threshold, then the subroutine 330 may move to the frequency of glucose testing sub-routine 410. After displaying the message in step 334, the sub-routine 330 may move to the frequency of blood glucose testing sub-routine 410.

Figure 13 shows a flow chart for the frequency of blood glucose testing sub-routine 410, which may include obtaining a number of blood glucose measurements over a total time period, as shown in step 411. Next, an average number of blood glucose concentration measurements per day or per week may be calculated, as shown in step 412. In step 413, the average number of average number of blood glucose concentration measurements per unit time is compared to a pre-determined threshold. A message may be displayed indicating that the average number of blood glucose concentration measurements per unit time is not sufficient if the average number of blood glucose concentration measurements per unit time is less than a pre-determined threshold, as shown in step 414. If the average number of blood glucose concentration measurements per unit time is not less than a pre-determined threshold, the sub-routine 410 may move to the adequacy of pre-meal testing sub-routine 420. After displaying the message in step 414, the sub-routine 410 may move to the adequacy of pre-meal testing sub-routine 420. In one embodiment, the pre-determined threshold may range from about 3 measurements per week to about 15 measurements per week.

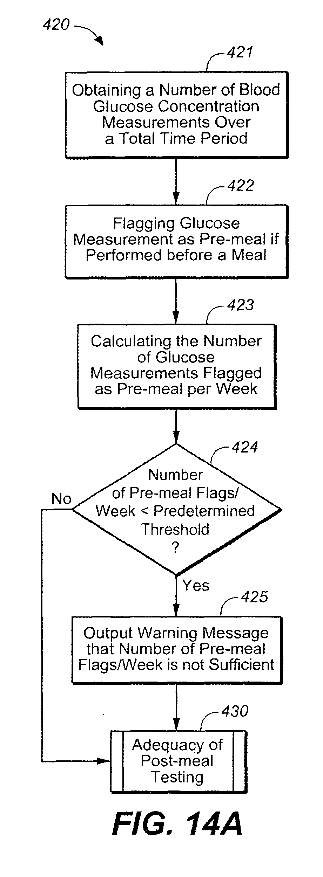

Figure 14A shows a flow chart for the adequacy of pre-meal testing sub-routine 420, which may include obtaining a number of blood glucose measurements over a total time period, as shown in step 421. Next, the blood glucose concentration measurement may be flagged as pre-meal if the blood glucose concentration measurement was performed before a meal, as shown in step 422. A number of blood glucose concentration measurements per week that are flagged as pre-meal can be determined, as shown in step 423. In step 424, the number of blood glucose concentration measurements flagged as pre-meal per week is compared to a pre-determined threshold. A warning message may be displayed if the number of blood glucose concentration measurements per week that are flagged as pre-meal is less than a pre-determined threshold, as shown in step 425. In one embodiment, the pre-determined threshold may range from about 3 pre-meal flags per week to about 7 pre-meal flags per week. However, it should be noted that the appropriate threshold is one that can be set by the physician or automatically or semi-automatically via a suitable algorithm by taking into account the average number of tests per day or per week, the pattern of testing being used, and the pattern of testing recommended by the physician. If the number of blood glucose concentration measurements per week that are flagged as pre-meal is not less than a pre-determined threshold, then the sub-routine 420 may move to the adequacy of post-meal testing sub-routine 430. After displaying the message in step 425, the sub-routine 420 may move to the adequacy of post-meal testing sub-routine 430. In other instances, where the patient is a type 2 diabetic, who as a group usually tests before meals, the message 425 may be dispensed with entirely. In an alternative embodiment, however, step 425 may include a message asking the user or patient to test or measure their blood glucose level more often, in the future, during a prescribed or determined time period as compared to any comparable prior time periods.

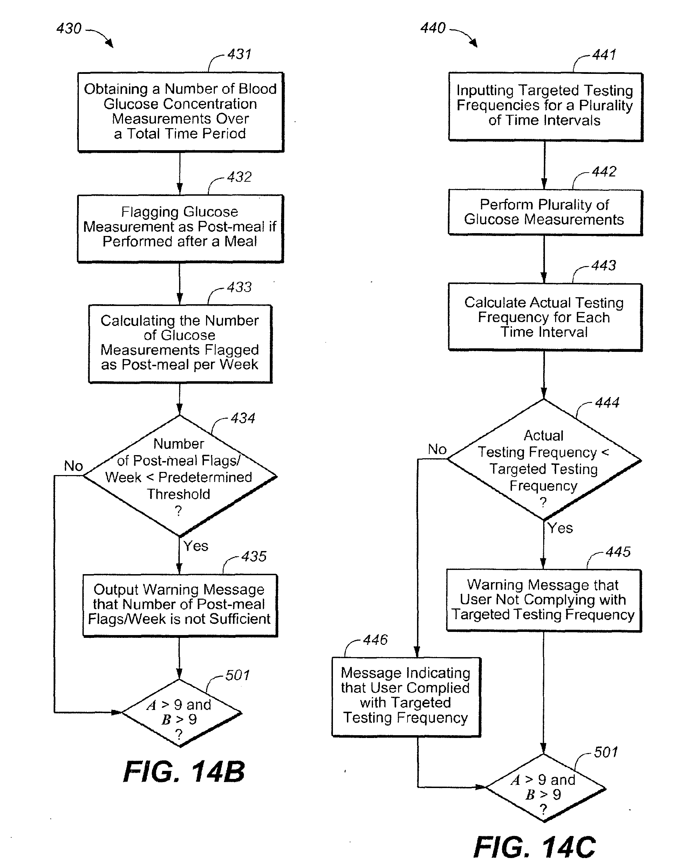

Figure 14B shows a flow chart for the adequacy of post-meal testing sub-routine 430, which may include obtaining a number of blood glucose measurements over a total time period, as shown in step 431. Next, the blood glucose concentration measurement may be flagged as post-meal if the blood glucose concentration measurement was performed after a meal, as shown in step 432. A number of blood glucose concentration measurements per week that are flagged as post-meal can be determined, as shown in step 433. In step 434, the number of blood glucose concentration measurements flagged as post-meal per week is compared to a pre-determined threshold. A warning message may be displayed if the number of blood glucose concentration measurements per week that are flagged as post-meal is less than a pre-determined threshold, as shown in step 435. However, it should be noted that the appropriate threshold is one that can be set by the physician or automatically or semi-automatically via a suitable algorithm by taking into account the average number of tests per day or per wee, the pattern of testing being used, and the pattern of testing recommended by the physician. In one embodiment, the pre-determined threshold may range from about 3 post-meal flags per week to about 7 post-meal flags per week. If the number of blood glucose concentration measurements per week that are flagged as post-meal is not less than a pre-determined threshold, then the sub-routine 430 may move to the step 501, where A indicates pre-meal testing frequency and B indicates post-meal testing frequency. After displaying the message in step 435, the sub-routine 430 may move to the step 501.

In an alternative embodiment, the adequacy of blood glucose testing sub-routine 440 may be performed after the adequacy of post-meal testing sub-routine 430. Figure 14C shows a flow chart for the adequacy of blood glucose testing sub-routine 440 that helps a physician determine a user's compliance in performing a sufficient number of blood glucose measurements. The adequacy of blood glucose testing sub-routine 440 may include inputting a plurality of targeted testing frequencies for a plurality of time intervals, as shown in step 441. The time intervals may include before breakfast, after breakfast, before lunch, after lunch, before dinner, after dinner, bedtime, and overnight. In one embodiment, a physician may input a targeted testing frequency for all of the time intervals to provide targeted goals for the user. Next, a number of blood glucose measurements may be performed at various time intervals, as shown in step 442. An actual testing frequency for each time interval may be calculated, as shown in step 443. In step 444, the actual testing frequency is compared to the targeted testing frequency. A warning message may be displayed if the actual testing frequency is less than the targeted testing frequency, as shown in step 445. If the actual testing frequency is not less than the targeted testing frequency, then a message indicating the user had complied with the targeted testing frequencies, as shown in step 446. After displaying the message in either step 445 or step 446, the sub-routine 440 may move to the step 501.

In another alternative embodiment, the testing/dosing pattern 400 may include sub-routines for recognizing patterns indicative of a pre-meal or post meal blood glucose measurements. A message may be displayed alerting a user that the most recently performed blood glucose measurement is pre-meal or post-meal based on past blood glucose measurements. The user may then be provided the option of flagging the blood glucose measurement with the appropriate flag.

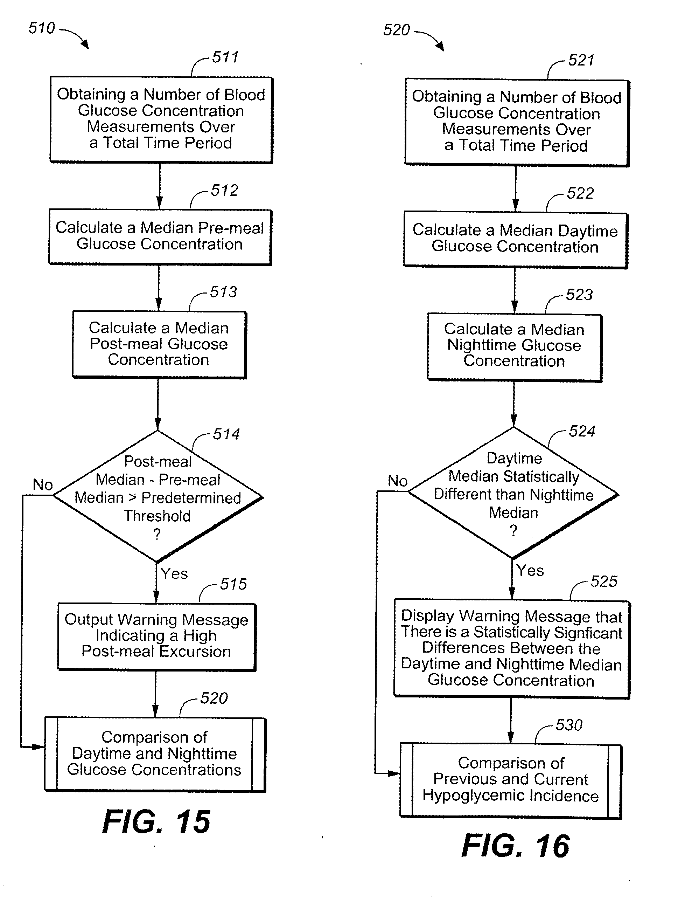

Figure 15 shows a flow chart for the comparison of pre-meal and post-meal blood glucose concentrations sub-routine 510, which may be used to determine whether there is a significant increase in blood glucose concentration after a user has ingested a meal. The comparison of pre-meal and post-meal blood glucose concentrations sub-routine 510 may include obtaining a number for blood glucose concentration measurements, as shown in step 511. Next, a median pre-meal blood glucose concentration and a median post-meal blood glucose concentration may be calculated, as shown in steps 512 and 513, respectively. Pre-meal and post-meal blood glucose concentrations may be defined as blood glucose concentration measurements that are flagged as pre-meal and post-meal, respectively. In step 514, the median pre-meal glucose concentration is subtracted from the median post-meal glucose concentration that results in a difference value. The difference value can be a clinically significant, statistically or both clinically and statistically significant value depending on the types of meal involved. A warning message indicating a high post-meal excursion if the difference value is greater than a pre-determined threshold, as shown in step 515. If the difference value is not greater than a pre-determined threshold, then the sub-routine 510 may move to the comparison of daytime and nighttime glucose concentrations sub-routine 520. After displaying the message in step 512, the sub-routine 510 may move to the comparison of daytime and nighttime glucose concentrations sub-routine 520. In one embodiment, the pre-determined threshold may be about 50 mg/dL. An embodiment may include a pre-condition where a warning message is not displayed unless there are greater than about nine measurements that are flagged as pre-meal and greater than about nine measurements that are flagged as post-meal.

Figure 16 shows a flow chart for the comparison of daytime and nighttime glucose concentrations sub-routine 520, which may be used to determine whether there is a significant difference in daytime and nighttime glucose concentrations. The comparison of daytime and nighttime glucose concentrations sub-routine 520 may include obtaining a number for blood glucose measurements over a total time period, as shown in step 521. Next, a median daytime glucose concentration and nighttime glucose concentration may be calculated, as shown in steps 522 and 523, respectively. Daytime may be a time interval ranging from about 6:00 A.M. to about 4:59 P.M. Nighttime may be a time interval ranging from about 5:00 P.M. to about 5:59 A.M. However, a daytime or nighttime period can be of any predetermined time periods, as selected by the physician or clinician. Next, a statistical test may be used to determine whether the daytime median is statistically significantly different from the nighttime median, as shown in step 524. If there are statistically significant differences, a warning message may be displayed indicating a difference between the daytime median and the nighttime median, as shown in step 525. If there is not a statistically significant difference, the sub-routine 520 may move to the comparison of previous and current hypoglycemic incidence sub-routine 530. After displaying the message in step 525, the sub-routine 520 may move to the comparison of previous and current hypoglycemic incidence sub-routine 530.

In one embodiment, the statistical test is a non-parametric statistical test. The non-parametric statistical test may be a Wilcoxon test or a Rank Sum test. The non-parametric test may include combining a plurality of glucose concentration measurements performed at the daytime time interval and at the nighttime time interval to form an aggregate of glucose concentration measurements. Next, the aggregate of glucose concentration measurements may be ranked in an ordinal order and a standardized rank sum $W_{std}$ may be calculated using an Equation 15.

$$W_{std} = \frac{W_d - \left\lfloor \frac{M_d * (N_d + N_n + 1)}{2} \right\rfloor}{\sqrt{\frac{N_d * N_n * (N_d + N_n + 1)}{12}}} \qquad \text{Eq. 15}$$

In Equation 15, the terms $W_{std}$ represents the standardized rank sum, $W_d$ represents a rank sum of the plurality of glucose concentration measurements performed at the daytime time interval, $N_d$ is the number of glucose concentration measurements for the daytime interval, and $N_n$ is the number of glucose concentration measurements for the nighttime interval. A warning message may be displayed indicating a statistically significant difference between the daytime median and the nighttime median if the standardized rank sum is greater than about 2. In one embodiment, the warning message may be displayed if the plurality of glucose concentration measurements performed at the daytime includes more than about 9 measurements. In another embodiment, the warning message may be displayed if the plurality of glucose concentration measurements performed at the nighttime includes more than about 9 measurements.

In another embodiment, the non-parametric test may be performed using a different equation if two or more glucose concentrations have a tie (i.e., have the same value). When two or more glucose concentrations have a tie, a standardized rank sum $W_{std}$ may be calculated using an Equation 16.

$$W_{std} = \frac{W_d - \left\lfloor \frac{N_d * (N_d + N_n + 1)}{2} \right\rfloor}{\left\lfloor \frac{N_d * N_n * (N_d + N_n + 1)}{12} \right\rfloor - \left\{ \frac{N_d * N_n}{12 * (N_d + N_n) * (N_d + N_n - 1)} * \sum_{j=1}^{g} (h_j - 1) * h_j * (h_j + 1) \right\}}$$

Eq. 16

In Equation 16, the therm $h_j$ represents a number of glucose concentration values within a tie, $j$ represents an index value associated with each group of glucose concentrations having a tie, and $g$ is a total number of ties. For example, if the blood glucose concentration values are 93, 93, 100, 100, 100, 104, 104, 104, 104 mg/dL, then $h_1 = 2$, $h_2 = 3$, $h_3 = 4$, and g = 3. A warning message may be displayed indicating a statistically significant difference between the daytime median and the nighttime median if the stantardized rank sum is greater than about 2.

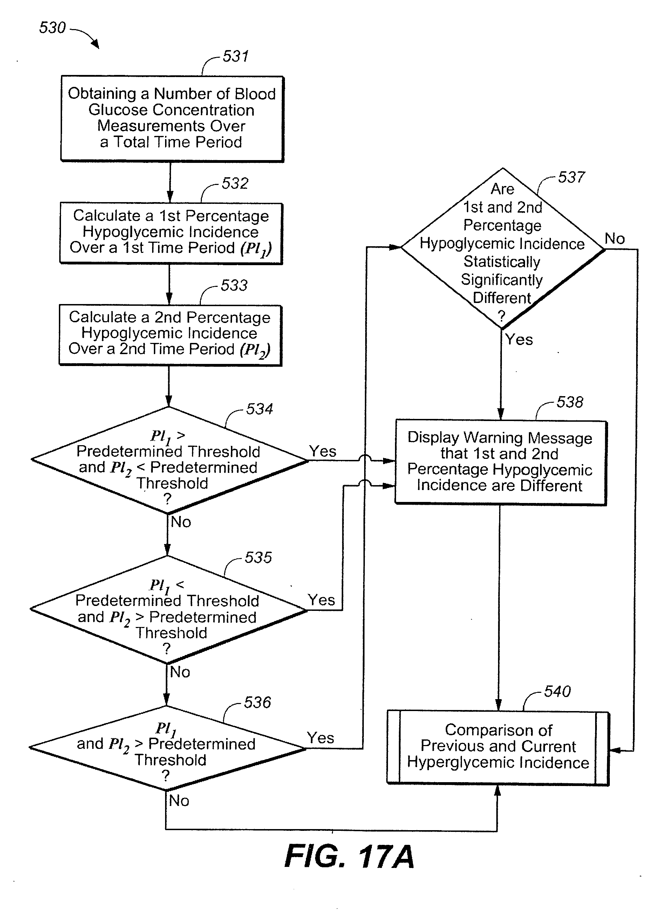

Figure 17A shows the comparison of previous and current hypoglycemic incidence sur-routine 530, which may indicate a statistical change from a current reporting period and a previous reporting period. A reporting period may be defined as the period between two successive device downloads for a given user. Note that the device does not have to be the same from doxnload to download.

The comparison of previous and current hypoglycemic incidence sub-routine 530 may include obtaining a number of blood glucose measurements over a total time period, as shown in step 531. Next, a first percentatge of hypoglycemic incidence $PI_1$ may be carculated over a first time period, as shown in step 532. A second percentage of hypoglycemic incidence $PI_2$ may be calculated over a second time period, as shown in step 533. The first time period may be a current time period and the second time period may be a previous time period.

In step 534, a calculation may be performed for determining if two conditions are achieved which are whether the first percentage of hypoglycemic incidence $PI_1$ is greater than a pre-determined threshold and whether the second percentage of hypoglycemic incidence $PI_2$ is less than a pre-determined threshold. If both of the above two conditions in step 534 are met, then the sub-routine 530 may show a warning message indicating a difference between the first percentage of hypoglycemic incidence $PI_1$ and the second percentage of hypoglycemic incidence $PI_2$, as shown in step 538. If at least one of the above two conditions are not met in step 534, then the sub-routine may move to step 535.

In the step 535, a calculation may be performed for determining if two conditions are achieved which are whether the first percentage of hypoglycemic incidence $PI_1$ is less than a pre-determined threshold and whether the second percentage of hypoglycemic incidence $PI_2$ is greater than a pre-determined threshold. If both of the above two conditions in step 535 are met, then the sub-routine 530 may show a warning message indicating a difference between the first percentage of hypoglycemic incidence $PI_1$ and the second percentage of hypoglycemic incidence $PI_2$, as shown in step 538. If at least one of the above two conditions are not met in step 535, then the sub-routine may move to step 536.

In the step 536, a calculation may be performed for determining whether the first percentage of hypoglycemic incidence $Pl_1$ and second percentage of hypoglycemic incidence $Pl_2$ are greater than a pre-determined threshold. If the above condition in step 536 is met, then the sub-routine 530 may perform a calculation for determining whether the first percentage of hypoglycemic incidence $Pl_1$ is statistically significantly different than the second percentage of hypoglycemic incidence $Pl_2$, as shown in step 537. If the above condition in step 536 is not met, then the sub-routine 530 may move to the comparison of previous and current hyperglycemic incidence sub-routine 540.

A calculation may be performed for determining whether the first percentage of hypoglycemic incidence $Pl_1$ is statistically significantly different than the second percentage of hypoglycemic incidence $Pl_2$, as shown in step 537. If a statistically significant difference is found between the first and second percentage of hypoglycemic incidence, a warning message may be displayed indicating the difference, as shown in step 538. If a statistically significant difference is not found between the first and second percentage of hypoglycemic incidence, the sub-routine 530 may move to the comparison of previous and current hyperglycemic incidence sub-routine 540. After displaying the message in the step 538, the sub-routine 530 may move to the comparison of previous and current hyperglycemic incidence sub-routine 540. In an embodiment of the comparison of previous and current hypoglycemic incidence sub-routine 530, the threshold may be about five percent or greater.

In one embodiment, a Z test may be used to determine whether the first percentage of hypoglycemic incidence is statistically significantly different than the second percentage of hypoglycemic incidence. The Z test may be performed using Equation 17.

$$Z = \frac{Pl_1 - Pl_2}{\sqrt{\left[\frac{Pl_1 * (1 - Pl_1)}{Nl_1}\right] + \left[\frac{Pl_2 * (1 - Pl_2)}{Nl_2}\right]}} \qquad \text{Eq. 17}$$

In Equation 17, the terms $Pl_1$ is the first percentage of hypoglycemic incidence, $Pl_2$ is the second percentage of hypoglycemic incidence, $Nl_1$ is the number of substantially hypoglycemic blood glucose concentration measurements that occur during the first time period, and $Nl_2$ is the number of substantially hypoglycemic blood glucose concentration measurements that occur during the second time period. A warning message may be displayed indicating a statistically significant difference between the first and second percentage of hypoglycemic incidence if Z is greater than about 2. In one embodiment, the warning message may be displayed if the number of substantially hypoglycemic blood glucose concentration measurements that occur during the first or second time period is greater than about 27.

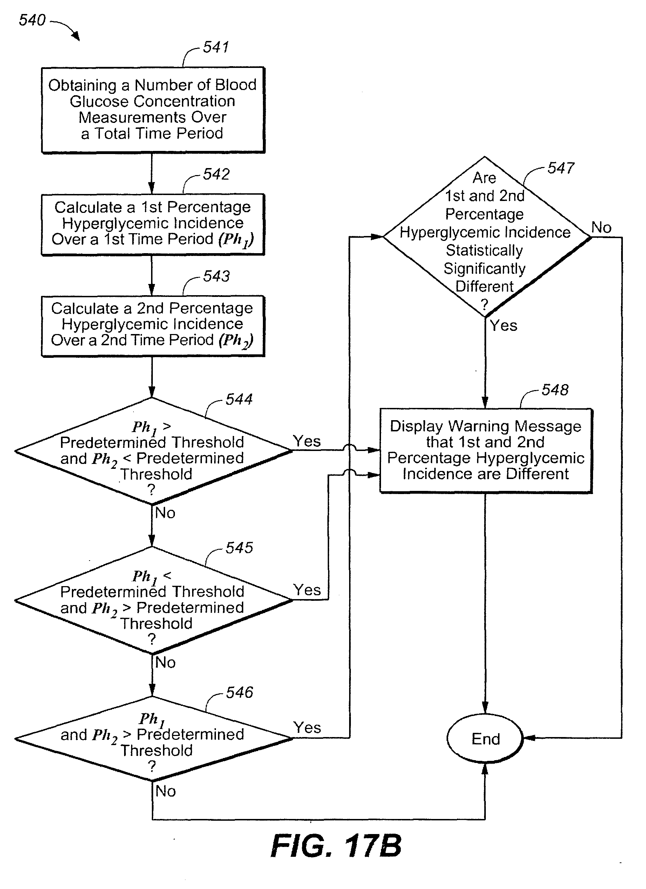

Figure 17B shows the comparison of previous and current hyperglycemic incidence sub-routine 540, which may indicate a statistical change from a current reporting period and a previous reporting period. A reporting period may be defined as the period between two successive device downloads for a given user. Note that the device does not have to be the same from download to download.

The comparison of previous and current hyperglycemic incidence sub-routine 540 may include obtaining a number of blood glucose measurements over a total time period, as shown in step 541. Next, a first percentage of hyperglycemic incidence $Ph_1$ may be calculated over a first time period, as shown in step 542. A second percentage of hyperglycemic incidence $Ph_2$ may be calculated over a second time period, as shown in step 543. The first time period may be a current time period and the second time period may be a previous time period.

In step 544, a calculation may be performed for determining if two conditions are achieved which are whether the first percentage of hyperglycemic incidence $Ph_1$ is greater than a pre-determined threshold and whether the second percentage of hyperglycemic incidence $Ph_2$ is less than a pre-determined threshold. If both of the above two conditions in step 544 are met, then the sub-routine 540 may show a warning message indicating a difference between the first percentage of hyperglycemic incidence $Ph_1$ and the second percentage of hyperglycemic incidence $Ph_2$, as shown in step 548. If at least one of the above two conditions are not met in step 544, then the sub-routine may move to step 545.

In the step 545, a calculation may be performed for determining if two conditions are achieved which are whether the first percentage of hyperglycemic incidence $Ph_1$ is less than a pre-determined threshold and whether the second percentage of hyperglycemic incidence $Ph_2$ is greater than a pre-determined threshold. If both of the above two conditions in step 545 are met, then the sub-routine 540 may show a warning message indicating a difference between the first percentage of hyperglycemic incidence $Ph_1$ and the second percentage of hyperglycemic incidence $Ph_2$, as shown in step 548. If at least one of the above two conditions are not met in step 545, then the sub-routine may move to step 546.

In the step 546, a calculation may be performed for determining whether the first percentage of hyperglycemic incidence $Ph_1$ and second percentage of hyperglycemic incidence $Ph_2$ are greater than a pre-determined threshold. If the above condition in step 546 is met, then the sub-routine 540 may perform a calculation for determining whether the first percentage of hyperglycemic incidence $Ph_1$ is statistically significantly different than the second percentage of hyperglycemic incidence $Ph_2$, as shown in step 547. If the above condition in step 546 is not met, then the sub-routine 540 may move to the end.

A calculation may be performed for determining whether the first percentage of hyperglycemic incidence $Ph_1$ is statistically significantly different than the second percentage of hyperglycemic incidence $Ph_2$, as shown in step 547. If a statistically significant difference is found between the first and second percentage of hyperglycemic incidence, a warning message may be displayed indicating the difference, as shown in step 548. If a statistically significant difference is not found between the first and second percentage of hyperglycemic incidence, the sub-routine 540 may move to the end. After displaying the message in the step 548, the sub-routine 540 may move to the end. In an embodiment of the comparison of previous and current hyperglycemic incidence sub-routine 540, the threshold may be about fifty percent or greater.

In one embodiment, a Z test may be used to determine whether the first percentage of hyperglycemic incidence is statistically significantly different than the second percentage of hyperglycemic incidence. The Z test may be performed using Equation 18.

$$Z = \frac{Ph_1 - Ph_2}{\sqrt{\left[\frac{Ph_1 * (1 - Ph_1)}{Nh_1}\right] + \left[\frac{Ph_2 * (1 - Ph_2)}{Nh_2}\right]}} \qquad \text{Eq. 18}$$

In Equation 18, the terms $Ph_1$ is the first percentage of hyperglycemic incidence, $Ph_2$ is the second percentage of hyperglycemic incidence, $Nh_1$ is the number of substantially hyperglycemic blood glucose concentration measurements that occur during the first time period, and $Nh_2$ is the number of substantially hyperglycemic blood glucose concentration measurements that occur during the second time period. A warning message may be displayed indicating a statistically significant difference between the first and second percentage of hyperglycemic incidence if $Z$ is greater than about 2. In one embodiment, the warning message may be displayed if the number of substantially hyperglycemic blood glucose concentration measurements that occur during the first or second time period is greater than about 27.

It should be noted here that while the glucose concentration in a patient is preferably obtained via the patient's blood for various exemplary embodiments, other physiological fluids from the patient can be utilized to provide a determination of glucose level such as, for example, interstitial fluid. Accordingly, it is intended that the word "glucose" (whether used herein alone or in conjunction with the word "blood," as in "blood glucose" or "glucose") to define not only glucose concentration or value present in blood but also in other biological fluids such as, for example, glucose concentration in interstitial fluid.

By virtue of the disclosure and illustrations provided herein, applicants have provided a communication medium to assist in diabetes management. The communication medium, as shown in Figure 18, includes a first display area D1 that has second, third, fourth, and fifth display areas D2, D3, D4, and D5, respectively, in the first display area,. The second display area D2 has identification information of a patient, whereas the third display area D3 has a plurality of textual messages, and the fourth display area D4 includes a graphical chart indicative of a variability of the glucose concentration over a predetermined time period of the patient, and other suitable information in graphical or textual format. The fifth display area D5 has a graphical chart of units of insulin taken over time for the patient. In the exemplary embodiments, the first display area D1 includes a display selected from any one of a video display monitor, a light projector, a sheet of paper, a hologram, an audio representation of the patient's variability trends (e.g., an automated voice response to the patient stating "high-variability between the hours of 3 A.M. and 9 A.M.), or combinations thereof.

The third display area D3 includes display of information for at least one of but are not limited to (a) incidence of hypoglycemia (b) incidence of hyperglycemia (c) blood glucose variability, (d) overcorrection, (e) differential between night time glucose concentration versus daytime glucose concentration, and (f) comparative analysis of glycemic state of the patient for a time period or at different time periods. In particular, the incidence of hypoglycemia includes (i) incidence of hypoglycemia by time period, (ii) incidence of hypoglycemia by day of the week, (iii) incidence of pre-meal hypoglycemia, and (iv) incidence of post-meal hypoglycemia. In other words, the third display area has a plurality of textual messages indicative of glycemic status of the patient including hypoglycemia, hyperglycemia, or excessive blood glucose variability.

Further, the incidence of hypoglycemia includes a textual indication of hypoglycemic incidence that includes a calculated percentage of hypoglycemic events within a predetermined time period, which is provided whenever the calculated percentage is greater than about 5% or a textual indication that all glucose readings are hypoglycemic is provided whenever the calculated percentage is 100%, otherwise no textual indication of hypoglycemic incidence is provided. A textual display of an indication of higher hypoglycemia in a specific time period is provided whenever a statistical correlation is determined between a time slot and a hypoglycemic event and there is an indication of hypoglycemic incidence. A textual display of an indication of higher hypoglycemia in a specific day of the week is provided whenever a statistical correlation is determined between the day of the week and a hypoglycemic event and there is a textual indication of hypoglycemic incidence. A textual display of pre-meal hypoglycemic events is provided whenever there are more than about 5% of pre-meal glucose readings marked as pre-meal glucose readings within a predetermined time period. Conversely, a textual display of post-meal hypoglycemic events is provided whenever there are more than about 5% of glucose readings marked as post-meal glucose readings within a predetermined time period.

The fourth display area D4 includes a graphical pattern of blood glucose variability about a median blood glucose value by at least one of time of day, of day in a week, of both time of day and day of week, or at different predetermined intervals. Although a graphical blood glucose variability pattern is shown in Figure 18 in relation to a specific day as spanning from 24 hours starting at about 12 A.M. to about 12 A.M., other relations can be also be viewed, as described earlier, in relation to day of a week, both time and day of week, specific date in a week or month, or over predetermined intervals, such as between physician's office visits or between different prescribed therapeutic regimens.

The communication medium also has the ability to provide information regarding incidence of hyperglycemia that includes but are not limited to (i) incidence of hyperglycemia by time period, (ii) incidence of hyperglycemia by day of the week, (iii) incidence of pre-meal hyperglycemia, and (iv) incidence of post-meal hyperglycemia. In particular, the incidence of hyperglycemia includes a textual indication of hyperglycemic incidence that includes a calculated percentage of hyperglycemic events within a predetermined time period is provided whenever the calculated percentage is greater than about 15% or a textual indication that all glucose readings are hyperglycemic is provided whenever the calculated percentage is 100%, otherwise no textual indication of hyperglycemic incidence is provided. Whenever a statistical correlation is determined between a time slot and a hyperglycemic event and there is a textual indication of hyperglycemic incidence, a textual display of an indication of higher hyperglycemia in a specific time period is provided. A textual display of an indication of higher hyperglycemia in a specific day of the week is provided whenever a statistical correlation is determined between the day of the week and a hyperglycemic event and there is a textual indication of hyperglycemic incidence. Whenever there is more than about 5% of glucose readings marked as post-meal glucose readings within a predetermined time period, a textual display of pre-meal hyperglycemic events is provided. And whenever there is more than about 5% of glucose readings marked as pre-meal glucose readings within a predetermined time period, a textual display of post-meal hyperglycemic events is also provided. Although 5% has been selected as a threshold, other values can be utilized depending on the therapeutic regimen prescribed by a physician, such as, for example, 10% or 15%.

The communication medium also has the ability to provide information relating to glucose variability, including, but not limited to (i) glucose variability range, (ii) possible rebound from hypoglycemia to hyperglycemia, (iii) incidence of possible overcorrection from hyperglycemia to hypoglycemia, or (iv) blood glucose variability associated with a specified indicator such as, for example, a specific time period during a day, a plurality of time periods in a day, a specified day in a week, a plurality of specified days in a week, pre-meal tests in a specific time period during a day, frequency of glucose measurements (i.e., testing) for pre-meal test for a specified day of the week, frequency of glucose measurements (i.e., testing) for pre-meal test for specified days of the week, glucose testing frequency having post-meal tests in a specific time period during a day, frequency of glucose measurements (i.e., testing) for post-meal test for a specified day of the week, or frequency of glucose measurements (i.e., testing) for post-meal test for specified days of the week.. A textual display indicative of high blood glucose variability is provided whenever a calculated blood glucose variability of a patient within a predetermined time period is about or greater than about a selected value, such as, for example, any value from about 30 mg/dL to about 90 milligrams per deciliter of glucose. A textual display indicative of a possibility of hypoglycemia to hyperglycemia rebound is provided whenever there is a change from a hyperglycemic event to a hypoglycemic event within a predetermined time period of less than about 4 hours. Whenever there is a change from a hypoglycemic event to a hyperglycemic event within a predetermined time period of less than about 4 hours, a textual display indicative of hyperglycemia to hypoglycemia rebound is provided.

Additionally, the communication medium can also provide information relating to differentials between pre and post meal data. Specifically, a textual indication of a calculated difference between pre-meal and post-meal medians within a reporting period is provided when the calculated difference is greater than about a selected value, such as, for example, any value from about 30 mg/dL to about 90 mg/dL, and preferably about 50 mg/dL.

Further, the communication medium can also provide information relating to hypo or hyper glycemic trends including a textual indication of one of an upward hypoglycemic trend or downward hypoglycemic trend based on a number of hypoglycemic measurement for two or more time periods, and a total number of glucose measurements for all of the time periods, as described earlier. Conversely, a textual indication of one of an upward hyperglycemic trend or downward hyperglycemic trend based on a number of hypoglycemic measurement for two or more time periods, and a total number of glucose measurements for all of the time periods. As used herein, the term "textual" is intended to cover not only text type representations but also numerical values, symbols (moving or stationary), charts, holograms, graphs, or combinations thereof.

Applicants have also, by virtue of the description and illustrations provided herein, provided for a computer program to provide diabetes management information to a user, which may include a clinician or a diabetic patient. The computer program includes a user interface, business object module, and a diabetes management rules engine, illustrated here in Figure 1 and described earlier. The diabetes rule management engine generates a plurality of textual pattern recognition messages based on a plurality of data inputs relating to blood glucose of a patient, including a graphical chart indicative of a blood glucose variability of the glucose concentration over a predetermined time period of the patient.

The plurality of textual messages may include information for at least one of but are not limited to (a) incidence of hypoglycemia (b) incidence of hyperglycemia (c) blood glucose variability, (d) overcorrection, (e) differential between night time glucose concentration versus daytime glucose concentration, and (f) comparative analysis of hypoglycemic or hyperglycemic trends. In particular, the incidence of hypoglycemia includes (i) incidence of hypoglycemia by time period, (ii) incidence of hypoglycemia by day of the week, (iii) incidence of pre-meal hypoglycemia, and (iv) incidence of post-meal hypoglycemia. Further, the incidence of hypoglycemia includes a textual indication of hypoglycemic incidence that includes a calculated percentage of hypoglycemic events within a predetermined time period is provided whenever the calculated percentage is greater than about 5% or a textual indication that all glucose readings are hypoglycemic is provided whenever the calculated percentage is 100%, otherwise no textual indication of hypoglycemic incidence is provided. A textual display of an indication of higher hypoglycemia in a specific time period is provided whenever a statistical correlation is determined between a time slot and a hypoglycemic event and there is an indication of hypoglycemic incidence. A textual display of an indication of higher hypoglycemia in a specific day of the week is provided whenever a statistical correlation is determined between the day of the week and a hypoglycemic event and there is such indication of hypoglycemic incidence. A textual display of pre-meal hypoglycemic events is provided whenever there are more than about 5% of glucose readings marked as pre-meal glucose readings within a predetermined time period. Conversely, a textual display of post-meal hypoglycemic events is provided whenever there are more than about 5% of glucose readings marked as post-meal glucose readings within a predetermined time period.

The computer program also has the ability to provide information regarding incidence of hyperglycemia that includes but are not limited to (i) incidence of hyperglycemia by time period, (ii) incidence of hyperglycemia by day of the week, (iii) incidence of pre-meal hyperglycemia, and (iv) incidence of post-meal hyperglycemia. In particular, the incidence of hyperglycemia includes a textual indication of hyperglycemic incidence that includes a calculated percentage of hyperglycemic events within a predetermined time period is provided whenever the calculated percentage is greater than about 15% or a textual indication that all glucose readings are hyperglycemic is provided whenever the calculated percentage is 100%, otherwise no textual indication of hyperglycemic incidence is provided. A textual display of an indication of higher hyperglycemia in a specific time period is provided whenever a statistical correlation is determined between a time slot and a hyperglycemic event and there is a textual indication of hyperglycemic incidence. Similarly, a textual display of an indication of higher hyperglycemia in a specific day of the week is provided whenever a statistical correlation is determined between the day of the week and a hyperglycemic event and there is indication of hyperglycemic incidence. Additionally, a textual display of pre-meal hyperglycemic events is provided whenever there are more than about 5% of glucose readings marked as pre-meal glucose readings within a predetermined time period. A textual display of post-meal hyperglycemic events is provided whenever there are more than about 5% of glucose readings marked as post-meal glucose readings within a predetermined time period.

The computer program also has the ability to provide information relating to glucose variability, including, but not limited to (i) glucose variability range, (ii) a possibility of hypoglycemia to hyperglycemia rebound, (iii) incidence of possible overcorrection from hyperglycemia to hypoglycemia, or (iv) blood glucose variability associated with a specified indicator such as, for example, a specific time period during a day, a plurality of time periods in a day, a specified day in a week, a plurality of specified days in a week, pre-meal tests in a specific time period during a day, frequency of glucose measurements (i.e., testing) for pre-meal test for a specified day of the week, frequency of glucose measurements (i.e., testing) for pre-meal test for specified days of the week, glucose testing frequency having post-meal tests in a specific time period during a day, frequency of glucose measurements (i.e., testing) for post-meal test for a specified day of the week, or frequency of glucose measurements (i.e., testing) for post-meal test for specified days of the week. A textual display indicative of high blood glucose variability is provided whenever a calculated blood glucose variability of a patient within a predetermined time period is about or greater than about 90 milligram per deciliter. Whenever there is a change from a hyperglycemic event to a hypoglycemic event within a predetermined time period less than about 4 hours a textual display indicative of a possibility of hypoglycemia to hyperglycemia rebound is provided. Similarly, a textual display indicative of hyperglycemia to hypoglycemia rebound is provided whenever there is a change from a hypoglycemic event to a hyperglycemic event within a predetermined time period less than about 4 hours.

Additionally, the computer program can also provide information relating to differential between pre and post meal data. Specifically, a textual indication of a calculated difference between pre-meal and post-meal medians within a reporting period is provided when the calculated difference is greater than about a predetermined value such as, for example, any value from about 30 mg/dL to about 90 mg/dL, and preferably about 50 mg/dL of glucose.

Further, the computer program can also provide information relating to hypo glycemic or hyper glycemic trends including a textual indication of one of an upward hypoglycemic trend or downward hypoglycemic trend based on a number of hypoglycemic measurement for two or more time periods, and a total number of glucose measurements for all of the time periods, as described earlier. Conversely, a textual indication of one of an upward hyperglycemic trend or downward hyperglycemic trend based on a number of hypoglycemic measurement for two or more time periods, and a total number of glucose measurements for all of the time periods. Set forth below in Table 1 are examples of various pattern recognition textual messages that can be provided to a clinician or user in managing diabetes:

**TABLE 1- Exemplary Pattern Recognition Messages**

| Message Nos. | Pattern Recognition Messages |
|---|---|
| 01 | Average number of glucose tests per week is |
| 02 | Average number of glucose tests per week flagged as pre-meal is |
| 03 | Average number of glucose tests per week flagged as post-meal is |
| 04 | _____% of values are hypoglycemic. |
| 05 | Higher incidence of hypoglycemia present for: |
| 06 | _____% of pre-meal values are hypoglycemic. |
| 07 | _____% of post-meal values are hypoglycemic. |
| 08 | All glucose readings are hyperglycemic. |
| 09 | _____% of values are hyperglycemic. |
| 10 | Higher incidence of hyperglycemia present for: |
| 11 | Higher incidence of hyperglycemia present for: |
| 12 | _____% of pre-meal values are hyperglycemic. |
| 13 | _____% of post-meal values are hyperglycemic. |
| 14 | High variability present. |
| 15 | Rebound from Low to High. |
| 16 | Overcorrection from High to Low. |
| 17 | The difference between pre and post-meal medians is: |
| 18 | The difference between daytime and nighttime glucose readings is significant. |
| 19 | Downward trend in the percentage of hypoglycemic values. |
| 20 | Downward trend in the percentage of hyperglycemic values. |
| 21 | . Note that no glucose tests were found in some time slots. |
| 22 | . Note that no glucose tests were found on certain days. |
| 23 | Nighttime readings are lower than daytime readings. |
| 24 | Daytime readings are lower than nighttime readings. |
| 25 | Upward trend in hypoglycemic events compared to the previous reporting period. |
| 26 | Downward trend in hypoglycemic events compared to the previous reporting period. |
| 27 | Upward trend in hyperglycemic events compared to the previous reporting period. |
| 28 | Downward trend in hyperglycemic events compared to the previous reporting period. |
| 29 | _____% of values are hypoglycemic. |
| 30 | Higher incidence of hypoglycemia present for: Note that no glucose tests were found in some time slots. |
| 31 | Higher incidence of hypoglycemia present for: Note that no glucose tests were found on certain days. |

(continued)

| Message Nos. | Pattern Recognition Messages |
|---|---|
| 32 | _____% of pre-meal values are hypoglycemic. |
| 33 | _____% of post-meal values are hypoglycemic. |
| 34 | _____% of values are hyperglycemic. |
| 35 | Higher incidence of hyperglycemia present for: Note that no glucose tests were found in some time slots. |
| 36 | Higher incidence of hyperglycemia present for: _____. Note that no glucose tests were found on certain days. |
| 37 | _____% of pre-meal values are hyperglycemic. |
| 38 | _____% of post-meal values are hyperglycemic. |

The computer program via the diabetes management rules engine also correlates (a) a median of glucose concentration values during a temporal period and (b) a median of test times during the temporal period to define a data point on a graph having glucose values and test times. Further, the computer program via the diabetes management rule engine correlates (i) a median of insulin doses over a temporal period and (ii) a median of dosage time during the temporal period to define a data point on a graph having insulin doses and dosage times. As used herein, the temporal period includes, but is not limited to at least one of a specific time period during a day, a plurality of time periods in a day, a specified day in a week, or a plurality of specified days in a week. In particular, the temporal period may also include at least one of testing incidence or glucose testing frequency having pre-meal tests in a specific time period during a day, frequency of glucose measurements (i.e., testing) for pre-meal test for a specified day of the week, frequency of glucose measurements (i.e., testing) for pre-meal test for specified days of the week, testing incidence having post-meal tests in a specific time period during a day, frequency of glucose measurements (i.e., testing) for post-meal test for a specified day of the week, or frequency of glucose measurements (i.e., testing) for post-meal test for specified days of the week.

It is believed that one advantage of the various embodiments is the ability of the process (which includes processes, machine, system or method) to transform patient's data (e.g., blood glucose level, insulin injection or type of insulin, carbohydrates intakes etc.) so as to provide useful, concrete and tangible results such as, for example, patterns that are statistically or clinically significant in managing diabetes. For example, the system transform blood glucose data of a patient into textual patterns using statistical analysis to provide simple and direct explanation of various patterns rather than complicated charts and graphs of the same. Referring to Figure 18, it can be seen that various "data patterns" such as, for example, percentage of data falling within hypoglycemic or hyperglycemic state, blood glucose variability, overcorrection, and differential between day and night blood glucose values are provided in clear and concise information for a busy clinician or user without the necessity of complicated graphs or tables. Other data can be presented in a graphical format to provide trends to a clinician such as, for example, pre-meal test time, post-meal test time, and overall target and median glucose values. A pre-meal test time may be a time of a glucose measurement performed before eating a meal and a post-meal test time may be a time of a glucose measurement performed after eating a meal. And although blood glucose variability information can be provided in as simple text, shown here in display area D3, it is sometimes more informative to utilize a graphical format to convey trends in blood glucose variability, shown here in display area D4. As such, a graphical chart over time is provided that shows the blood glucose variability of the 1st and 4th interquartile ranges about a median glucose curve over time. Correlation between median blood glucose values (area D4) and median insulin injections (area D5) over the same time period (e.g., "TP", time of day, day of week, pre or post meal over a set time period) can be provided to a clinician interested in seeing a generalized effect of insulin or types of insulin on blood glucose value.

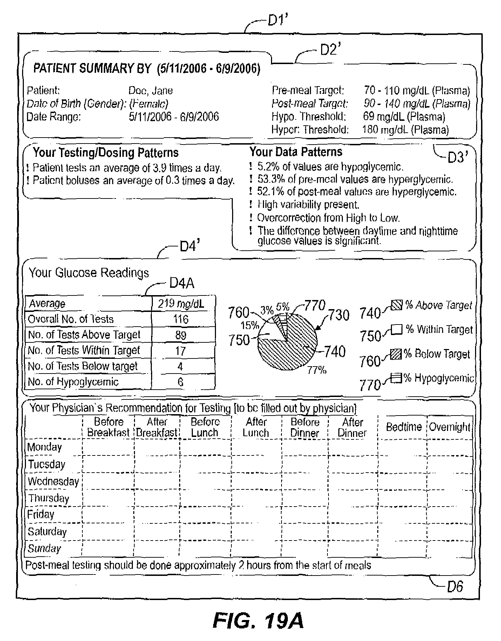

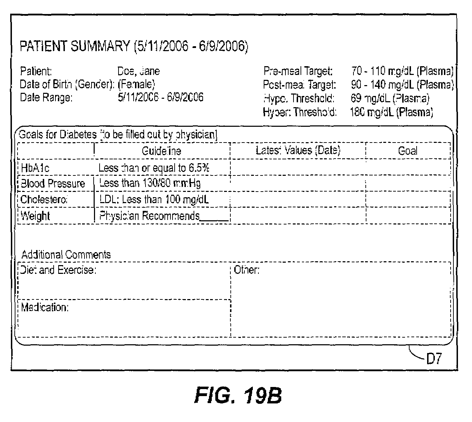

Referring to Figures 19A and 19B, the exemplary system also provides an alternative display format D1' for use by the patient after the visit with the physician or clinician. In particular, as shown in Figure 19A, the display area D2' and D3' provide the same patient information as display area D1 of Figure 18. Display area D4', however, provides for information that are believed to be more readily understood by a diabetic patient. For example, display area D4' shows the average blood glucose with the overall number of glucose measurements. The system further categorizes in table D4A the data into the number of tests above a target (which is set by the physician), below target and hypoglycemic. The system further provides a graphical chart 730 to demonstrate to the patient the data in table D4A in percentages. In the preferred embodiments, the graphical chart 730 is a pie chart showing the percentage (of a total number of tests in a predetermined time period) above target 740; percentage within target 750; percentage below target; and percentage hypoglycemic. A physician or clinician recommendation for testing is provided in area D6. Other information such as goals or targets is provided in display area D7.

While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially. Therefore, to the extent there are variations of the invention, which are within the spirit of the disclosure or equivalent to the inventions found in the claims, it is the intent that this patent will cover those variations as well.

**Claims**

1. A computer program to provide diabetes management information to a user, the computer program comprising:

    a user interface;
    a database module having a plurality of data inputs relating to blood glucose of a patient;
    a business object module in communication with the database module; and
    a diabetes management rules engine that generates a plurality of textual messages to the user interface based on the database module and the business object module, the textual messages including statistically significant patterns from the patient's data indicative of at least excessive variability by at least one of time of day, by day in a week, both by time of day and day of week, or at different time intervals.

2. The computer program of Claim 1, wherein the textual messages comprises a display of at least one of (a) incidence of hypoglycemia (b) incidence of hyperglycemia (c) overcorrection, (d) differential between night time blood glucose concentration versus daytime blood glucose concentration, and (e) comparative analysis of hypoglycemic or hyperglycemic trends.

3. The computer program of Claim 2, wherein the incidence of hypoglycemia includes (i) incidence of hypoglycemia by time period, (ii) incidence of hypoglycemia by day of the week, (iii) incidence of pre-meal hypoglycemia, and (iv) incidence of post-meal hypoglycemia.

4. The computer program of Claim 2, wherein the incidence of hypoglycemia comprises a textual indication of hypoglycemic incidence that includes a calculated percentage of hypoglycemic events within a predetermined time period being provided whenever the calculated percentage is greater than about 5% or a textual indication that all blood glucose readings are hypoglycemic is provided whenever the calculated percentage is 100%, otherwise no textual indication of hypoglycemic incidence is provided.

5. The computer program of Claim 4, wherein a textual messages comprises a display of an indication of higher hypoglycemia in a specific time period being provided whenever a statistical correlation is determined between a time slot and a hypoglycemic event and there is a textual indication of hypoglycemic incidence.

6. The computer program of Claim 4, wherein a textual message comprises a display of an indication of higher hypoglycemia in a specific day of the week is provided whenever a statistical correlation is determined between the day of the week and a hypoglycemic event and there is a textual indication of hypoglycemic incidence.

7. The computer program of Claim 2, wherein a textual message comprises a display of an incidence or frequency of pre-meal hypoglycemic events being provided whenever there are more than about 5% of blood glucose readings are hypoglycemic and are marked as pre-meal blood glucose readings within a predetermined time period.

8. The computer program of Claim 2, wherein a textual message comprises a display of post-meal hypoglycemic events being provided whenever there are more than about 5% of blood glucose readings are hypoglycemic and are marked as post-meal blood glucose readings within a predetermined time period.

9. The computer program of Claim 2, wherein the incidence of hypoglycemia includes (i) incidence of hyperglycemia by time period, (ii) incidence of hyperglycemia by day of the week, (iii) incidence of pre-meal hyperglycemia, and (iv) incidence of post-meal hyperglycemia.

10. The computer program of Claim 9, wherein the incidence of hyperglycemia comprises a textual indication of hyperglycemic incidence that includes a calculated percentage of hyperglycemic events within a predetermined time period, which is provided whenever the calculated percentage is greater than about 15% or a textual indication that all blood glucose readings are hyperglycemic is provided whenever the calculated percentage is 100%, otherwise no textual indication of hyperglycemic incidence is provided.

11. The computer program of Claim 10, wherein a textual message comprises a display of an indication of higher hyperglycemia in a specific time period being provided whenever a statistical significance is determined between a time slot and a hyperglycemic event and there is a textual indication of hyperglycemic incidence.

12. The computer program of Claim 10, wherein a textual message comprises a display of an indication of higher hyperglycemia in a specific day of the week being provided whenever a statistical significance is determined between the day of the week and a hyperglycemic event and there is a textual indication of hyperglycemic incidence.

13. The computer program of Claim 9, wherein a textual message comprises a display of an incidence of pre-meal hyperglycemic events being provided whenever there is more than about 5% of blood glucose readings marked as pre-meal blood glucose readings within a predetermined time period.

14. The computer program of Claim 9, wherein a textual message comprises a display of an incidence of post-meal hyperglycemic events being provided whenever there is more than about 5% of blood glucose readings marked as post-meal blood glucose readings within a predetermined time period.

15. The computer program of Claim 2, wherein the variability comprises (i) overall variability, (ii) hypoglycemia to hyperglycemia rebound, and (iii) incidence of hyperglycemia to hypoglycemia.

16. The computer program of Claim 15, wherein a textual message comprises a display indicative of high variability being provided whenever a calculated variability of a patient within a predetermined time period is about or greater than about 90 milligram per deciliter.

17. The computer program of Claim 15, wherein a textual message comprises a display indicative of hypoglycemia to hyperglycemia rebound being provided whenever there is a change from a hyperglycemic event to a hypoglycemic event within a predetermined time period less than about 4 hours.

18. The computer program of Claim 15, wherein a textual message comprises a display indicative of hyperglycemia to hypoglycemia rebound being provided whenever there is a change from a hypoglycemic event to a hyperglycemic event within a predetermined time period less than about 4 hours.

19. The computer program of Claim 2, wherein the differential comprises a textual indication of a calculated difference between pre-meal and post-meal medians within a reporting period being provided when the calculated difference is greater than about 50mg/dL.

20. The computer program of Claim 2, wherein the comparative analysis hypoglycemic trend comprises a textual indication of one of an upward hypoglycemic trend or downward hypoglycemic trend based on a based on a number of hypoglycemic measurement for two or more time periods, and a total number of blood glucose measurements for all of the time periods.

21. The computer program of Claim 2, wherein the comparative analysis hyperglycemic trend comprises a textual indication of one of an upward hypoglycemic trend or downward hypoglycemic trend based on a number of hypoglycemic measurement for two or more time periods, and a total number of blood glucose measurements for all of the time periods.

22. The computer program of Claim 1, wherein the diabetes management rule engine correlates (a) a median of blood glucose concentration values during a temporal period and (b) a median of test incidences during the temporal to define a data point on a two-dimensional coordinate graph having blood glucose values and test times.

23. The computer program of Claim 22, wherein the diabetes management rule engine correlates (i) a median of insulin injection quantities over a temporal period and (ii) a median of injection incidences during the temporal period to define a data point on a two-dimensional coordinate graph having insulin injection quantities and injection times.

26

24. The computer program of Claim 23, wherein the temporal period comprises at least one of a specific time period during a day, a plurality of time periods in a day, a specified day in a week, or a plurality of specified days in a week.

25. The computer program of Claim 24, wherein the temporal period comprises at least one of testing incidences having pre-meal tests in a specific time period during a day, testing incidences having pre-meal tests in a specified day of a week, testing incidences having pre-meal tests in a plurality of days in a week, testing incidences having post-meal tests in a specific time period during a day, testing incidences having post-meal tests in a specified day of a week, or testing incidences having post-meal tests in a plurality of days in a week.

**FIG. 1**

Adequacy of
Glucose Testing
440

Post-meal
Flag Prompt
450

40

Incidence of Hypoglycemia
110

Frequency of
Glucose Testing
410

Adequacy of
Post-meal Testing
430

Hypoglycemic Pattern
by Time Slot
120

Adequacy of
Pre-meal Testing
420

Pre-meal
Flag Prompt
460

Hypoglycemic Pattern
by Day of Week
130

Testing/
Dosing
Pattern
400

Incidence of
Hypoglycemia by Meal
140

Physiological
Pattern
800

Hypoglycemia
Pattern
100

Carbohydrate
Intake
Pattern
700

Diabetes Management
Rules Engine

Hyperglycemia
Pattern
200

Incidence of
Hyperglycemia
210

Hyperglycemic Pattern
by Time Slot
220

Hyperglycemic Pattern
by Day of Week
230

Insulin
Pattern
600

Comparative
Pattern
500

Variability
Pattern
300

Incidence of
Hyperglycemia by Meal
240

Comparison of Pre-
meal and Post-meal
Glucose Concentrations
510

Comparison of
Previous and
Current
Hypoglycemic
Incidence
530

Glucose Variability
Range
310

Comparison of
Daytime and Nighttime
Glucose Concentrations
520

Over-correction for
Hypoglycemia
320

Comparison of
Previous and
Current
Hyperglycemic
Incidence
540

Over-correction for
Hyperglycemia
330

Pre-meal Flag
Prompt by Insulin
610

Post-meal Flag
Prompt by Insulin
620

**FIG. 2**

**FIG. 3A**

**FIG. 3**

FIG. 3A | FIG. 3B

*410*
Frequency of Glucose Testing

*420*
Adequacy of Pre-meal Testing

*330*
Over-correction for Hyperglycemia

*430*
Adequacy of Post-meal Testing

*320*
Over-correction for Hypoglycemia

*501*
$A > 9$ and $B > 9$ ?
No
Yes

*510*
Comparison of Pre-meal and Post-meal Glucose Concentrations

*520*
Comparison of Daytime and Nighttime Glucose Concentrations

*530*
Comparison of Previous and Current Hypoglycemic Incidence

*540*
Comparison of Previous and Current Hyperglycemic Incidence

No

Yes
*310*
Glucose Variability Range

END

**FIG. 3B**

**FIG. 4A**

**FIG. 4B**

## FIG. 4C

| | Outcome 1 (e.g. Hypoglycemic) | | Outcome 2 (e.g., Not Hypoglycemic) | | | | |
|---|---|---|---|---|---|---|---|
| | Observed | Expected | Observed | Expected | Row Total | SE | Z test |
| Condition 1 (e.g., day of week) | $L_1$ | $L_{1,pre} = \dfrac{\sum_{i=1}^{n} L_i}{\sum_{i=1}^{n} N_i} * N_1$ | $L_1'$ | $L_{1,pre}' = \dfrac{\sum_{i=1}^{n} L_i'}{\sum_{i=1}^{n} N_i} * N_1$ | $N_1 = L_1 + L_1'$ | $SE_1 = \sqrt{\dfrac{1}{N_1} * L_{1,pre} * (N_1 - L_{1,pre})}$ | $Z_1 = \dfrac{(L_1 - L_{1,pre})}{SE_1}$ |
| Condition 2 | $L_2$ | $L_{2,pre} = \dfrac{\sum_{i=1}^{n} L_i}{\sum_{i=1}^{n} N_i} * N_2$ | $L_2'$ | $L_{2,pre}' = \dfrac{\sum_{i=1}^{n} L_i'}{\sum_{i=1}^{n} N_i} * N_2$ | $N_2 = L_2 + L_1'$ | $SE_2 = \sqrt{\dfrac{1}{N_2} * L_{2,pre} * (N_2 - L_{2,pre})}$ | $Z_2 = \dfrac{(L_2 - L_{2,pre})}{SE_2}$ |
| Condition 3 | $L_3$ | $L_{3,pre} = \dfrac{\sum_{i=1}^{n} L_i}{\sum_{i=1}^{n} N_i} * N_3$ | $L_3'$ | $L_{3,pre}' = \dfrac{\sum_{i=1}^{n} L_i'}{\sum_{i=1}^{n} N_i} * N_3$ | $N_3 = L_3 + L_3'$ | $SE_3 = \sqrt{\dfrac{1}{N_3} * L_{3,pre} * (N_3 - L_{3,pre})}$ | $Z_3 = \dfrac{(L_3 - L_{3,pre})}{SE_3}$ |
| ...... | ...... | ...... | ...... | ...... | ...... | ...... | ...... |
| ...... | ...... | ...... | ...... | ...... | ...... | ...... | ...... |
| ...... | ...... | ...... | ...... | ...... | ...... | ...... | ...... |
| Condition $n$ | $L_n$ | $L_{n,pre} = \dfrac{\sum_{i=1}^{n} L_i}{\sum_{i=1}^{n} N_i} * N_n$ | $L_n'$ | $L_{n,pre}' = \dfrac{\sum_{i=1}^{n} L_i'}{\sum_{i=1}^{n} N_i} * N_n$ | $N_n = L_n + L_n'$ | $SE_n = \sqrt{\dfrac{1}{N_n} * L_{n,pre} * (N_n - L_{n,pre})}$ | $Z_n = \dfrac{(L_n - L_{n,pre})}{SE_n}$ |

**FIG. 5**

**FIG. 6**

210 ⌐

**213**

Obtain a Number of Blood Glucose Concentration Measurements Over a Total Time Period

↓

**214**

Calculating a Percent Hyperglycemic Incidence *(Ph)*

↓

**215**

$Ph >$ Predetermined Threshold ?

No → 

**240**

Incidence of Hyperglycemia by Meal

Yes ↓

**216**

Output Warning Message Indicating a High Incidence of Hyperglycemia

↓

**211**

$Ph = 100$ ?

**FIG. 7A**

220 ⌐

**221**

Obtain a Number of Blood Glucose Concentration Measurements Over a Total Time Period

↓

**222**

Determine Number of Hyperglycemic Concentration Measurements by Time Slot

↓

**223**

Perform $\chi^2$ Test

↓

**224**

Is Calc $\chi^2 >$ Table $\chi^2$ ?

No →

Yes ↓

**225**

Perform $Z$ Test for Each Time Slot

↓

**226**

Is $Z_i > 2$ ?

No → 

**228**

$N > 46$ ?

Yes ↓

**227**

Output Warning Message Indicating Which Time Slots have a High Incidence of Hyperglycemia

**FIG. 7B**

35

230 ⟍　　231

Obtain a Number of Blood
Glucose Concentration
Measurements Over
a Total Time Period

232

Determine Number of
Hyperglycemic Concentration
Measurements by Day of Week

233

Perform $\chi^2$ Test

234

Is
Calc $\chi^2$ > Table $\chi^2$
?　　No

Yes

235

Perform $Z$ Test for
Each Day of the Week

236

Is $Z_i$ > 2
?　　No

Yes

240

Incidence of
Hyperglycemia
by Meal

237

Output Warning
Message Indicating
Which Day of the
Week has a High
Incidence of
Hyperglycemia

**FIG. 8**

240 ⟍　　241

Obtain a Number of Blood Glucose
Concentration Measurements Over
a Total Time Period

242

Determine Number of Blood
Glucose Concentration
Measurements with a Pre-meal
Tag $(A)$ and Post-meal Tag $(B)$

243

Determine Number of
Hypoglycemic Glucose
Measurements Having a Pre-
meal Tag $(H_A)$ and Post-meal
Tag $(H_B)$

244

Calculate Percent Hyperglycemic
Incident Having a Pre-meal Tag
$(Ph_A)$ and Post-meal Tag $(Ph_B)$

245

$Ph_A$ or
$Ph_B$ >
Threshold
?　　No

Yes

301

Sufficient
Number of
Measurements to
Determine
Variability
?

246

Output Warning
Message Indicating
High Incidence of
Hyperglycemia at
Either Pre-meal
and/or Post-meal

**FIG. 9**

310

311

Obtain a Number of Blood
Glucose Measurements Over
a Total Time Period

312

Rank All Measurements

313

Assign Upper and
Lower Ranking for
Interquartile Range

314

Upper
Ranking - Lower
Threshold
?

No

Yes

315

Output Warning Message
Indicating an Incidence of
High Variability

320

Over-correction
for
Hypoglycemia

**FIG. 10**

320

321

Obtain a Number of Blood
Glucose Measurements Over
a Total Time Period

322

Locating a 1st Blood
Glucose Concentration that
is Hypoglycemic

323

Any
Blood Glucose
Concentrations Measured
About 30-240 Minutes After 1st
Blood Glucose Concentration
> Predetermined
Threshold
?

No

Yes

324

A Possible Presence of
Over-correction for
Hypoglycemia

330

Over-correction
for
Hyperglycemia

**FIG. 11**

## FIG. 12

330 →

**331**
Obtaining a Number of Blood Glucose Measurements Over a Total Time Period

↓

**332**
Locating a 1st Blood Glucose Concentration that is Hyperglycemic

↓

**333**
Any Blood Glucose Concentrations Measured about 30-240 Minutes after 1st Blood Glucose Concentration < Predetermined Threshold ?

No / Yes

**334**
A Possible Presence of Over-correction for Hypoglycemia

↓

**410**
Frequency of Glucose Testing

**FIG. 12**

## FIG. 13

410 →

**411**
Obtaining a Number of Blood Glucose Concentration Measurements Over a Total Time Period

↓

**412**
Calculating an Average Number of Measurements per Week or Day

↓

**413**
Is Average Number of Measurements per Week or Day < Predetermined Threshold ?

No / Yes

**414**
Output Warning Message that Average Number of Glucose Measurements per Unit Time is Not Sufficient

↓

**420**
Adequacy of Pre-meal Testing

**FIG. 13**

420 —

421

Obtaining a Number of Blood
Glucose Concentration
Measurements Over
a Total Time Period

422

Flagging Glucose
Measurement as Pre-meal if
Performed before a Meal

423

Calculating the Number
of Glucose
Measurements Flagged
as Pre-meal per Week

424

Number
of Pre-meal Flags/
Week < Predetermined
Threshold
?

No

Yes

425

Output Warning Message
that Number of Pre-meal
Flags/Week is not Sufficient

430

Adequacy of
Post-meal
Testing

FIG. 14A

430 ⟍

431
Obtaining a Number of Blood Glucose Concentration Measurements Over a Total Time Period

432
Flagging Glucose Measurement as Post-meal if Performed after a Meal

433
Calculating the Number of Glucose Measurements Flagged as Post-meal per Week

434
Number of Post-meal Flags/ Week < Predetermined Threshold ?

No

Yes

435
Output Warning Message that Number of Post-meal Flags/Week is not Sufficient

501
$A > 9$ and $B > 9$ ?

**FIG. 14B**

440 ⟍

441
Inputting Targeted Testing Frequencies for a Plurality of Time Intervals

442
Perform Plurality of Glucose Measurements

443
Calculate Actual Testing Frequency for Each Time Interval

444
Actual Testing Frequency < Targeted Testing Frequency ?

No

Yes

445
Warning Message that User Not Complying with Targeted Testing Frequency

446
Message Indicating that User Complied with Targeted Testing Frequency

501
$A > 9$ and $B > 9$ ?

**FIG. 14C**

510 ⟍

511
Obtaining a Number of Blood
Glucose Concentration
Measurements Over
a Total Time Period

512
Calculate a Median Pre-meal
Glucose Concentration

513
Calculate a Median
Post-meal Glucose
Concentration

514
Post-meal
Median - Pre-meal
Median > Predetermined
Threshold
?

No

Yes

515
Output Warning Message
Indicating a High
Post-meal Excursion

520
Comparison of
Daytime and Nighttime
Glucose Concentrations

**FIG. 15**

520 ⟍

521
Obtaining a Number of Blood
Glucose Concentration
Measurements Over
a Total Time Period

522
Calculate a Median Daytime
Glucose Concentration

523
Calculate a Median
Nighttime Glucose
Concentration

524
Daytime
Median Statistically
Different than Nighttime
Median
?

No

Yes

525
Display Warning Message that
There is a Statistically Signficant
Differences Between the
Daytime and Nighttime Median
Glucose Concentration

530
Comparison of
Previous and Current
Hypoglycemic Incidence

**FIG. 16**

530 ─↘

531

Obtaining a Number of Blood
Glucose Concentration
Measurements Over
a Total Time Period

532

Calculate a 1st Percentage
Hypoglycemic Incidence
Over a 1st Time Period $(PI_1)$

533

Calculate a 2nd Percentage
Hypoglycemic Incidence
Over a 2nd Time Period $(PI_2)$

534

$PI_1 >$
Predetermined Threshold
and $PI_2 <$ Predetermined
Threshold
? — Yes

No

535

$PI_1 <$
Predetermined Threshold
and $PI_2 >$ Predetermined
Threshold
? — Yes

No

536

$PI_1$
and $PI_2 >$ Predetermined
Threshold
? — Yes

No

537

Are
1st and 2nd
Percentage
Hypoglycemic Incidence
Statistically
Significantly
Different
? — No

Yes

538

Display Warning Message
that 1st and 2nd
Percentage Hypoglycemic
Incidence are Different

540

Comparison of
Previous and Current
Hyperglycemic Incidence

**FIG. 17A**

540 ─

541

Obtaining a Number of Blood
Glucose Concentration
Measurements Over
a Total Time Period

542

Calculate a 1st Percentage
Hyperglycemic Incidence
Over a 1st Time Period $(Ph_1)$

543

Calculate a 2nd Percentage
Hyperglycemic Incidence
Over a 2nd Time Period $(Ph_2)$

544

$Ph_1 >$
Predetermined Threshold
and $Ph_2 <$ Predetermined
Threshold
?

Yes

No

545

$Ph_1 <$
Predetermined Threshold
and $Ph_2 >$ Predetermined
Threshold
?

Yes

No

546

$Ph_1$
and $Ph_2 >$ Predetermined
Threshold
?

Yes

No

547

Are
1st and 2nd
Percentage
Hyperglycemic Incidence
Statistically
Significantly
Different
?

No

Yes

548

Display Warning Message
that 1st and 2nd
Percentage Hyperglycemic
Incidence are Different

End

**FIG. 17B**

FIG. 18

D1'

D2'

**PATIENT SUMMARY BY (5/11/2006 - 6/9/2006)**

| | |
|---|---|
| Patient: | Doe, Jane |
| Date of Birth (Gender): (Female) | |
| Date Range: | 5/11/2006 - 6/9/2006 |

| | |
|---|---|
| Pre-meal Target: | 70 - 110 mg/dL (Plasma) |
| Post-meal Target: | 90 - 140 mg/dL (Plasma) |
| Hypo. Threshold: | 69 mg/dL (Plasma) |
| Hyper. Threshold: | 180 mg/dL (Plasma) |

**Your Testing/Dosing Patterns**

! Patient tests an average of 3.9 times a day.
! Patient boluses an average of 0.3 times a day.

**Your Data Patterns** D3'

! 5.2% of values are hypoglycemic.
! 53.3% of pre-meal values are hyperglycemic.
! 52.1% of post-meal values are hyperglycemic.
! High variability present.
! Overcorrection from High to Low.
! The difference between daytime and nighttime glucose values is significant.

D4'

**Your Glucose Readings**

D4A

| | |
|---|---|
| Average | 219 mg/dL |
| Overall No. of Tests | 116 |
| No. of Tests Above Target | 89 |
| No. of Tests Within Target | 17 |
| No. of Tests Below target | 4 |
| No. of Hypoglycemic | 6 |

760 3% 5% 770 730 740 ⬚ % Above Target
15%
750 750 ⬚ % Within Target
740
760 ⬚ % Below Target
77%
770 ⬚ % Hypoglycemic

**Your Physician's Recommendation for Testing [to be filled out by physician]**

| | Before Breakfast | After Breakfast | Before Lunch | After Lunch | Before Dinner | After Dinner | Bedtime | Overnight |
|---|---|---|---|---|---|---|---|---|
| Monday | | | | | | | | |
| Tuesday | | | | | | | | |
| Wednesday | | | | | | | | |
| Thursday | | | | | | | | |
| Friday | | | | | | | | |
| Saturday | | | | | | | | |
| Sunday | | | | | | | | |

Post-meal testing should be done approximately 2 hours from the start of meals

D6

**FIG. 19A**

PATIENT SUMMARY (5/11/2006 - 6/9/2006)

Patient:                        Doe, Jane            Pre-meal Target:     70 - 110 mg/dL (Plasma)
Date of Birth (Gender): (Female)                     Post-meal Target:    90 - 140 mg/dL (Plasma)
Date Range:                     5/11/2006 - 6/9/2006 Hypo. Threshold:     69 mg/dL (Plasma)
                                                     Hyper. Threshold:    180 mg/dL (Plasma)

Goals for Diabetes [to be filled out by physician]

|                | Guideline                     | Latest Values (Date) | Goal |
|----------------|-------------------------------|----------------------|------|
| HbA1c          | Less than or equal to 6.5%    |                      |      |
| Blood Pressure | Less than 130/80 mmHg         |                      |      |
| Cholesterol    | LDL: Less than 100 mg/dL      |                      |      |
| Weight         | Physician Recommends____      |                      |      |

Additional Comments

| Diet and Exercise: | Other: |
|--------------------|--------|
| Medication:        |        |

D7

**FIG. 19B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 68862907 A
- US 142903 A

### Non-patent literature cited in the description

- **J. SCHLICHTKRULL et al.** The M-Value, an Index of Blood-Sugar Control in Diabetics. *Acta Medica Scandinavia,* 1965, vol. 177, 95-93
- **F. JOHN SERVICE et al.** Mean Amplitude of Glycemic Excursions, a Measure of Diabetic Instability. *Diabetes,* September 1970, vol. 19 (9), 644-655
- **KOVATCHEV BP et al.** Methods for Quantifying Self-monitoring Blood Glucose Profile Exemplified by an Examination of Blood Glucose Pattern in Patients with Type I and Type 2 Diabetes. *Diabetes Technology and Therapeutics,* 2002, vol. 4, 295-303
- **RYAN EA et al.** Assessment of the Severity of Hypoglycemia and Glycemic Lability in Type 1 Diabetic Subjects Undergoing Islet Transplantation. *Diabetes,* 2004, vol. 53, 955-962
- **RODBARD, D.** Improved Methods for Calculating a ''Figure of Merit'' for Blood Glucose Monitoring Data. *Diabetes Technology Meeting,* November 2005
- **WOJCICKI, J.** J-Index, A New Proposition Of The Assessment Of Current Glucose Control In Diabetic Patients. *Horm Metab Res.,* 1995, vol. 27, 41-42
- **OTTO et al.** *Diabetes Care,* November 2006, vol. 29 (11), 2433-2438
- **HIRSCH IB.** Glycemic Variability: It's Not Just About A1C Anymore!. *Diabetes Technol Ther.,* 2005, vol. 7, 780-783
- **BROWNLEE M ; HIRSCH, I.B.** Glycemic variability: A Hemoglobin A1c - Independent Risk Factor For Diabetic Complications. *JAMA,* 2006, vol. 295 (14), 1707-1708
- **MONNIER L ; MAS E ; GINET C et al.** Activation Of Oxidative Stress By Acute Glucose Fluctuations Compared With Sustained Chronic Hyperglycemia In Patients With Type 2 Diabetes. *JAMA,* 2006, vol. 295, 1681-1687